# EUROPEAN PATENT APPLICATION

(11) **EP 2 390 248 A1**
(43) Date of publication of application: **30.11.2011**
(21) Application number: 10382140.1
(22) Date of filing: 24.05.2010
(51) Int. Cl.: C07D 211/22, C07D 211/24, C07D 401/12, C07D 405/12, C07D 409/06, A61K 31/4462, A61K 31/4465, A61K 31/4545, A61P 25/28

(54) **Flufenoxine derivatives for the treatment and prevention of amiloyd pathologies**

(71) Applicant: FAES FARMA, S.A., 48940 Lamiako - Leioa - Vizcaya (ES)
(72) Inventor: Ledo Gómez, Francisco, E-48940 Lamiako (Lejona) - Vizcaya (ES); Munoz, Munoz, Ana, E-48940 Lamiako (Lejona) - Vizcaya (ES)
(74) Representative: ABG Patentes, S.L.

(57) **Abstract**

The present invention is directed to a compound of formula (I) for use in a method to treat or ameliorate amyloid or tau pathologies, such as Alzheimer's disease, or symptoms thereof. The invention is also directed to new compounds of formula (I), of subformula (II), (III), (IV), or (V).

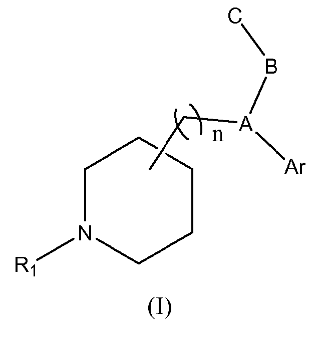

## Description

### FIELD OF THE INVENTION

The present invention relates to flufenoxine derivatives useful to treat amyloid or tau pathologies, such as Alzheimer's disease, and/or ameliorate symptoms thereof.

### STATE OF THE ART

Alzheimer's disease (AD) is the amyloid pathology most extensively studied, and the most common cause of dementia in the elderly that affects an estimated 15 million people worldwide and 40 percent of the population above 85 years. The disease is characterized by progressive loss of memory, speech and movement with a total incapacitation of the patient and eventually death.

No cure is currently available for amyloid pathologies. Today, medication therapy focuses, at best, on controlling the symptoms and their various stages. For example, mild to moderate AD can involve treatment with cholinesterase inhibitors such as Cognex(R) (tacrine), Aricept (donepezil), Exelon(R) (rivastigmine), or Razadyne(R) (galantamine). Whereas moderate to severe AD can be treated with Namenda(R) (memantine). These medications may help delay or prevent AD symptoms from becoming worse for a limited period of time.

Therefore, there is a need to provide further drugs to treat and/or ameliorate, amyloid pathologies, for example, AD. These drugs should preferably have improved properties, such as, for example, increased efficacy, less toxicity, improved bioavailability, etc.

When autopsied, studies of brain tissue have identified two major hallmarks of AD, the deposition of extracellular amyloid plaques intracellular and neurofibrillary tangles (NFT).

Amyloid plaque is primarily composed of aggregated amyloid-beta (Aβ) peptides, which are originated from proteolysis of the amyloid precursor protein (APP). Monomers of 40-amino acid (Aβ40) are much more prevalent than the aggregation-prone and damaging Aβ42 species. An imbalance between production and clearance causes Aβ to accumulate, and this excess may be the initiating factor in AD. This idea is strongly supported by the analysis of early-onset familial AD mutations and the successive validation in transgenic animal models. Thus, transgenic mice that express a mutant form of the human APP gene develop fibrillar amyloid plaques and Alzheimer's-like brain pathology with spatial learning deficits.

Further, it has been hypothesized that the APOE4 (Apolipoprotein E), the major genetic risk factor for AD, could lead to excess amyloid buildup in the brain before AD symptoms arise. Thus, Aβ deposition is believed to precede clinical AD.

Thus, amyloid buildup in the brain is directly linked to neurodegenerative diseases (Tebbenkamp AT, Borchelt DR. Methods Mol Biol. 2009;566:85-91), e.g. Alzheimer's disease (De Arai, T, Ikeda, K, Akiyama, H, Tsuchiya, K, Iritani, S, Ishiguro, K, Yagishita, S, Oda, T, Odawara, T, Iseki, E (2003) ACTA NEUROPATHOLOGICA 105: 489-498), Parkinson's disease (Burack MA, Hartlein J, Flores HP, Taylor-Reinwald L, Perlmutter JS, Cairns NJ Neurology 2010 Jan 5;74(1):77-84), Creutzfeldt-Jakob, or Lewy body dementia (Dupiereux I, Zorzi W, Quadrio I, Perret-Liaudet A, Kovacs GG, Heinen E, Elmoualij B. Cent Nerv Syst Agents Med Chem. 2009 Mar;9(1):2-11).

NFTs contain bundles of PHF (paired helical filaments), the major component of which is hyperphosphorylated tau, a microtubule associated protein. It has also been hypothesized that hyperphosphorylated tau begins to pair with other threads of tau. Eventually, they form neurofibrillary tangles inside nerve cell bodies. When this occurs, the microtubules disintegrate, collapsing the neuron's transport system. This may result first in malfunctions in biochemical communication between neurons and later in the death of the cells.

Therefore, tau hyperphosphorilation has also been linked to neurodegenerative pathologies (Armstrong, RA, Lantos, PL, Cairns, NJ (2005) Neuropatologia 25: 111-124; Avila, J, Lim, F, Moreno, F, Belmonte, C, Cuello, AC (2002) Neurobiologia Molecular 25: 213-231; Avila, J, Pérez, M, Lim, F, Gómez-Ramos, A, Hernández, F, Lucas, JJ (2004) Neurotoxicidad Investigación 6: 477-482), e.g. Alzheimer's disease (De Arai, T, et al (2003) ACTA NEUROPATHOLOGICA 105: 489-498), Parkinson's disease (Morishima-Kawashima, M, et al (2002) Journal of Neuroscience Research 70: 392-401), Pick's disease (Bird, T, et al (2003) Annals of Neurology 54: S29-S31), supranuclear progressive palsy (Berry, RW, et al (2004) JOURNAL OF NEUROCYTOLOGY 33: 287-295), DCB (Weeks, RA, et al (2003) MOVEMENT DISORDERS 18: 331-336), frontotemporal dementia (Binetti, G, et al (2003) NEUROSCIENCE LETTERS 338: 85-87), syndrome of the frontal lobe (M, Larsson, et al (2003) Journal of Neurology, Neurosurgery and Psychiatry 74: 867-871), Down's Sindrome (Barbiero, L, et al (2003) Experimental Neurology 182: 335-345), dementia (Bornebroek, M, et al (2004) Clinical Neuroscience Research 3: 349-361), frontotemporal mild cognitive impairment (de Mendonca, A, et al (2004) REVISTA DE LA ENFERMEDAD DE ALZHEIMER 6: 1-9), corticobasal degeneration (Graham, NL, et al (2003) TRASTORNOS DEL MOVIMIENTO 18: 1224-1232), progressive primary aphasia (Sobrido, MJ, et al (2003) NEUROLOGÍA 60: 862-864), amyotrophic lateral sclerosis (73. Lomen-Hoerth, C, et al (2002) The overlap of amyotrophic lateral sclerosis and frontotemporal dementia. NEUROLOGY 59: 1077-1079), multisystemic atrophy (Neumann, M, et al (2004) ACTA NEUROPATHOLOGICA 107: 489-496), progressive presenile dementia, bitemporal atrophia (Ostojic, J, et al (2004) GERIÁTRICA 17: 298-301), Creutzfeldt-Jakob (Shimamura, M, Uyama, et al (2003) MEDICINA INTERNA 42: 195-198), cerebral amyoid angiopathy (Weeks, RA, et al (2003) MOVEMENT DISORDERS 18: 331-336).

Thus, there is great interest in the search of compounds capable of inhibiting tau phosphorilation and/or inhibiting beta amyloid deposition, as they would be useful in the treatment of amyloid and tau pathologies.

W02005/105763 discloses the usefulness of a family of compounds as norepinephrine reuptake inhibitors, and as potential drugs for the treatment of Alzheimer's disease, characterized by a substituted morpholine ring.

Flufenoxine and salts thereof are known to inhibit serotonin and/or noradrenaline uptake, and are known to have anti-depressant properties. In Orjales et al J. Med. Chem, 2003, 46, 5512-5532 derivates of flufenoxine are described as being compounds with high affinity for the serotonin transporter (most of them also for the norepinephrine transporter), but no indication is given that they could be useful for the treatment of amyloid pathologies such as Alzheimer Disease.

US6518284 discloses compounds of formula (I), without providing any indication as to their usefulness for the treatment of amyloid pathologies such as Alzheimer Disease.

### BRIEF DESCRIPTION OF THE FIGURES

Figure 1: tau phosphorylation as the mean of 3 independent experiments of the relative level of tau-1 in soluble cell extracts obtained from cerebellar granule neurons treated with the indicated compounds. COMPOUND 6 is the reference compound
Figure 2: COMPOUND 3 ability to modify the secretion levels of the amyloid peptide (β1-40) of transgenic cell line CHO-APP-PS1
Figure 3: COMPOUND 10 ability to modify the secretion levels of the amyloid peptide (β-40) of transgenic cell line CHO-APP-PS1
Figure 4: COMPOUND 9(+) ability to modify the secretion levels of the amyloid peptide (β1-40) of transgenic cell line CHO-APP-PS
Figure 5: COMPOUND 4 ability to modify the secretion levels of the amyloid peptide (β1-40) of transgenic cell line CHO-APP-PS1
Figure 6: COMPOUND 3, COMPOUND 10 and COMPOUND 4 ability to modify the secretion levels of the peptide β1-40 in primary culture of cerebellar neurons derived from Tg-APP-PS1 transgenic mice
Figure 7: COMPOUND 10 ability to modify the secretion levels of the peptide β1-40 in primary culture of cerebellar neurons derived from Tg-APP-PS1 transgenic mice
Figure 8: COMPOUND 31 and COMPOUND 41 ability to modify the secretion levels of the peptide β1-40 in primary culture of cerebellar neurons derived from Tg-APP-PS1 transgenic mice
Figure 9: COMPOUND 34, COMPOUND 29 and COMPOUND 37 ability to modify the secretion levels of the peptide β1-40 in primary culture of cerebellar neurons derived from Tg-APP-PS1 transgenic mice
Figure 10: COMPOUND 2, COMPOUND 36 and COMPOUND 35 ability to modify the secretion levels of the peptide β1-40 in primary culture of cerebellar neurons derived from Tg-APP-PS1 transgenic mice
Figure 11: COMPOUND 5(-) and COMPOUND 5(+) ability to modify the secretion levels of the peptide β1**-**40 in primary culture of cerebellar neurons derived from Tg-APP-PS1 transgenic mice

### SUMMARY OF THE INVENTION

The inventors have discovered that some known and new flufenoxine derivatives can be surprisingly useful in the treatment of amyloid and tau pathologies, and show, for example, increased efficacy, less toxicity and/or improved bioavailability. The compounds are surprisingly effective. They have shown biological activity in both histopathological markers of amyloid and tau pathologies, amyloid deposition and Tau hyperphosphorylation. This dual activity could lead to drugs which could truly modify the course of amyloid or tau pathologies, such as AD.

Thus, according to a first aspect, the invention is directed to a compound of formula (I): 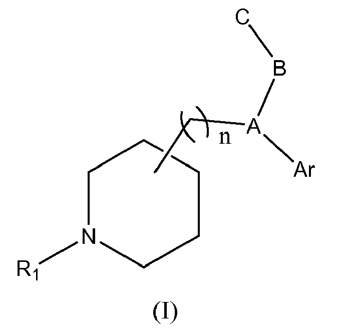 wherein:
R₁ is selected from the group consisting of hydrogen or -(CH₂)_{w}-(C=O)_{y}—(CH₂)ₓ-R₂, wherein R₂ is a C₁-C₆ alkyl group optionally substituted by -NH₂, -SH, -OH or C₃-C₈ cycloalkyl, or R₂ is a heterocyclyl group optionally substituted by a substituted or unsubstituted phenyl, or by a substituted or unsubstituted 5- or 6-membered heteroaryl group; w is an integer selected from 0, 1, 2 or 3; x is an integer selected from 0, 1, 2 or 3; and y is 0 or 1;
Ar is an optionally substituted phenyl or an optionally substituted 5- or 6-membered heteroaryl group;
when A is ―C(H)-; B is ―(CH₂)-O-, ―(CH₂)-S-, ―O- or ―S-; and C is an optionally substituted phenyl, an optionally substituted benyl or an optionally substituted naftyl group; or
when A is ―C(=)-; B is =C(H)-; and C is selected from the group consisting of ―OH, ―O(C₁-C₃-alkyl), and -O(C₇-C₁₀-arylalkyl), or is an optionally substituted phenyl or an optionally substituted naphtyl group; and
n is an integer selected from 0, 1 or 2;
or a pharmaceutically acceptable salt, stereoisomer and/or solvate thereof
for use in a method to treat or ameliorate amyloid or tau pathologies, or symptoms thereof.

Some of the derivatives having in common the usefulness in the treatment of amyloid or tau pathologies are new chemical compounds. Accordingly, a further aspect of the invention is a compound of formula (II), of formula (III), of formula (IV), or of formula (V)

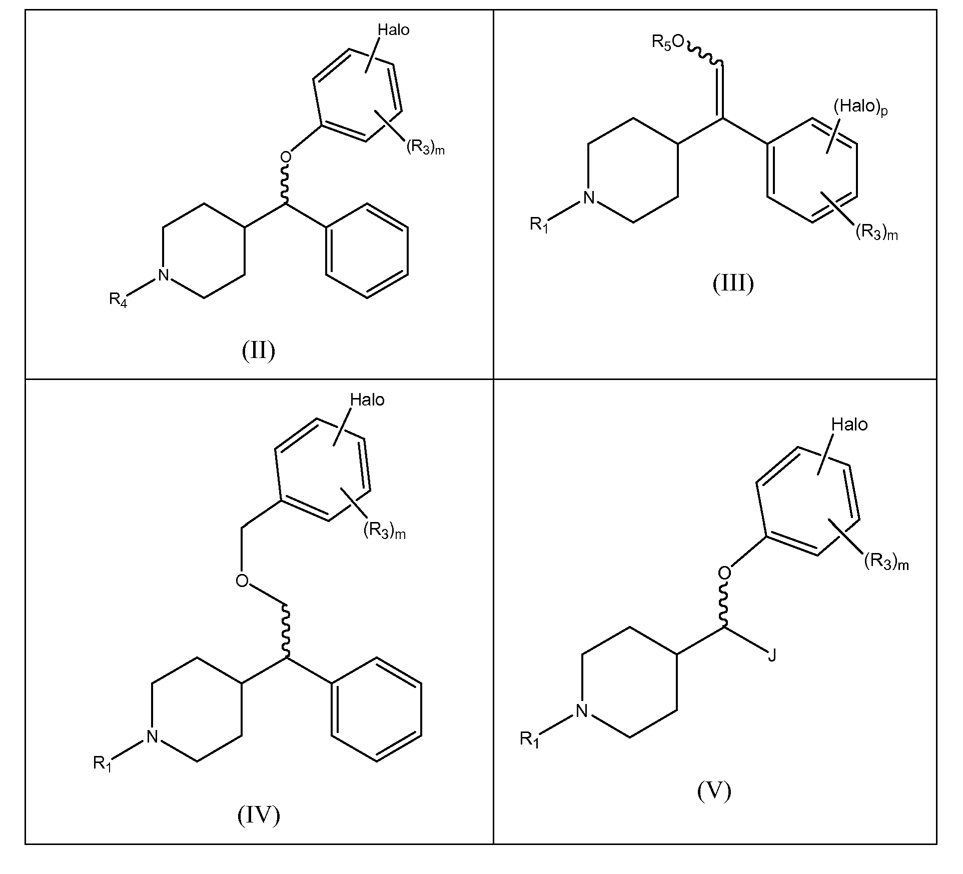

wherein in each case where appropriate
R₁ as defined above, preferably wherein R₁ is hydrogen or a C₁-C₆ alkyl group optionally substituted by -NH₂, -SH, -OH or C₃-C₈ cycloalkyl;
Ar is an optionally substituted phenyl or an optionally substituted 5- or 6-membered heteroaryl group;
R₄ is -(CH₂)_{w}-(C=O)_{y}—(CH₂)ₓ-R₂, wherein R₂ is a heterocyclyl group optionally substituted by a substituted or unsubstituted phenyl, or by a substituted or unsubstituted 5- or 6-membered heteroaryl group; w is an integer selected from 0, 1, 2 or 3; x is an integer selected from 0, 1, 2 or 3; and y is 0 or 1;
HeteAryl is an optionally substituted 5- or 6-membered heteroaryl group;
Halo is ―F, -Cl, -Br or ―I;
R₃ is selected from the group consisting of hydrogen, ―F, -Cl, -Br, ―I, C₁-C₃ alkyl, C₁-C₃ alkenyl, C₁-C₃ perfluoroalkyl, -O-C₁-C₃-alkyl, -O-C₁-C₃-alkenyl, -CN; and m is 1, 2 or 3; and
p is 0 or 1
or a pharmaceutically acceptable salt, stereoisomer and/or solvate thereof.

A further aspect of the invention is a derivative selected from the group consisting of:

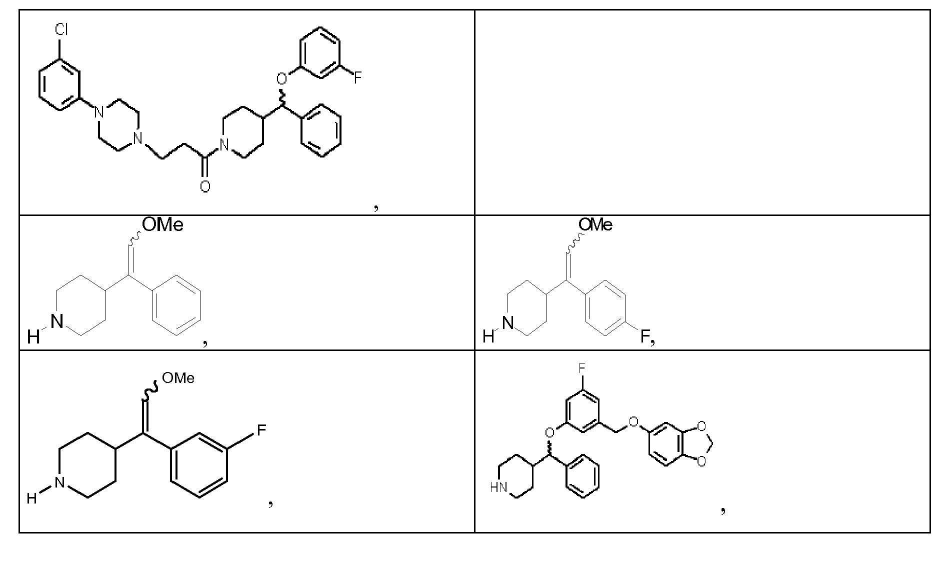

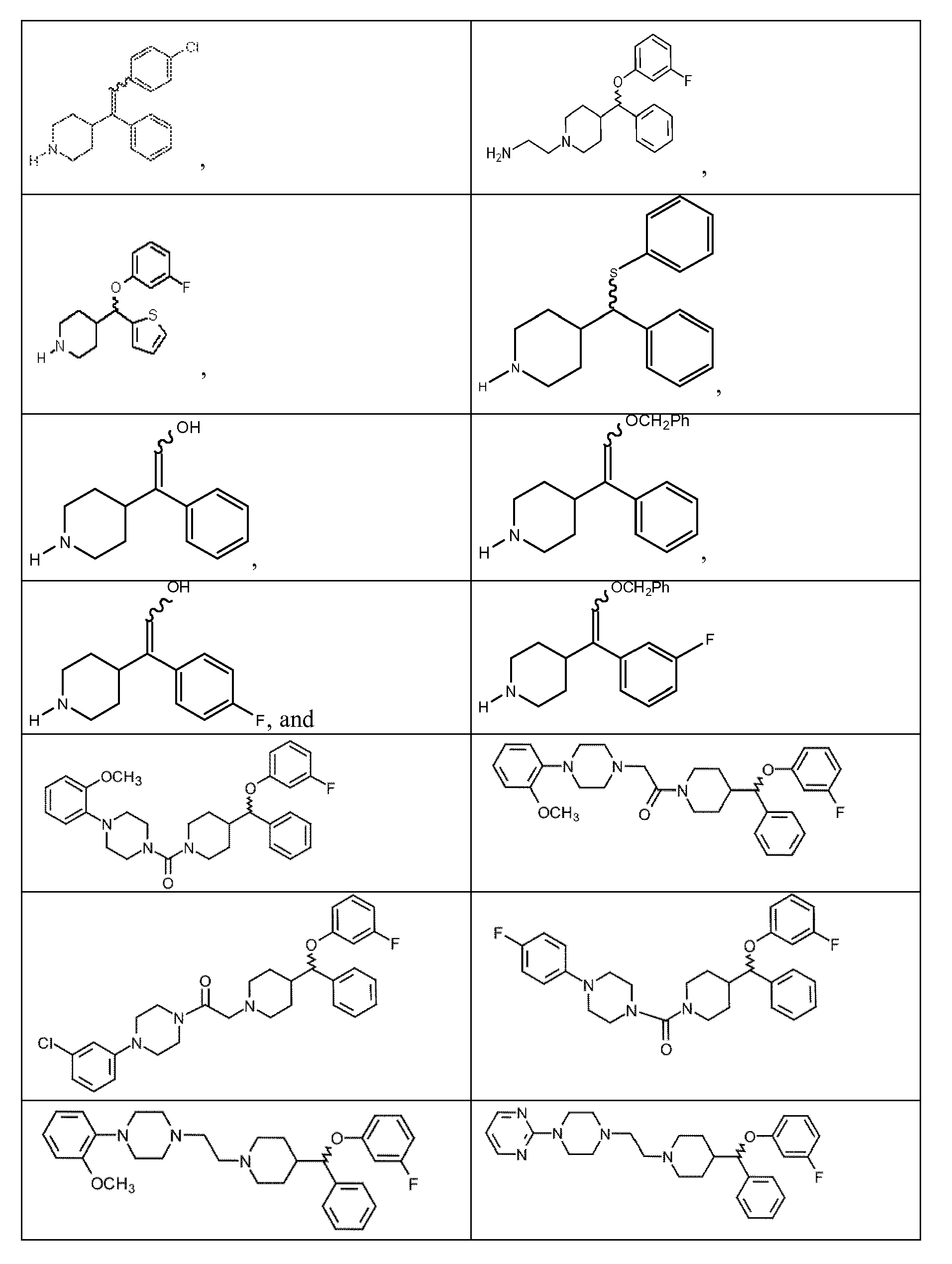

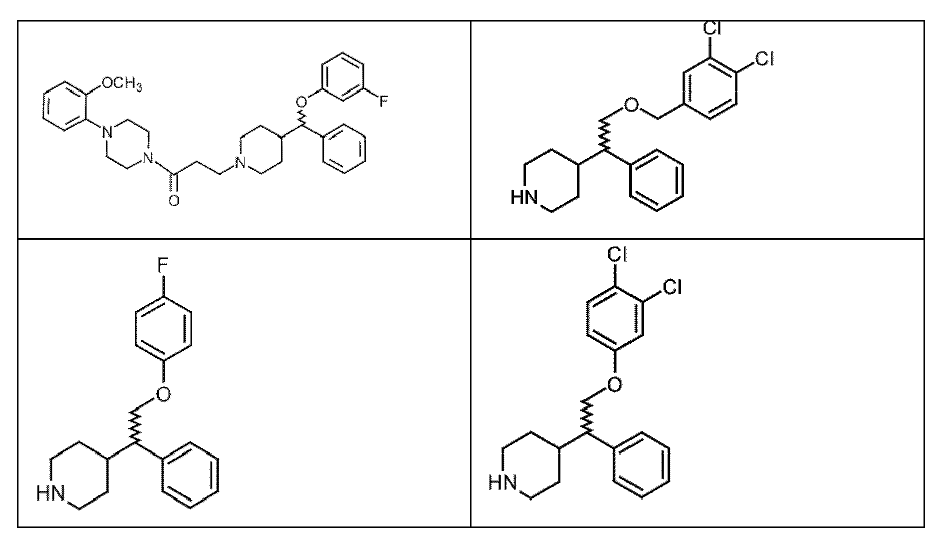

or a pharmaceutically acceptable salt, stereoisomer and/or solvate thereof.

A further aspect of the invention is a pharmaceutical composition comprising a compound of formula (II), (III), (IV), or (V) as defined above, or a derivative as defined above, or a pharmaceutically acceptable salt, stereoisomer and/or solvate thereof, and at least one pharmaceutically acceptable carrier.

A further aspect of the invention is a compound of formula (II), (III), (IV), or (V) as defined above, or a pharmaceutically acceptable salt, stereoisomer and/or solvate thereof for use as a medicament.

A further aspect of the invention is a compound of formula (II), (III), (IV), or (V) as defined above, or a pharmaceutically acceptable salt, stereoisomer and/or solvate thereof for use in a method to treat or ameliorate an amyloid or tau pathology or symptoms thereof.

### DETAILED DESCRIPTION OF THE INVENTION

In the context of the present invention, the following terms have the meaning detailed below:
The term "C₁₋₆ alkyl" or "C₁-C₆-alkyl" refers to a linear or branched hydrocarbon chain radical consisting of carbon and hydrogen atoms, containing no unsaturation, having between 1 and 6, preferably between 1 and 3 ("C₁₋₃ alkyl" or or "C₁-C₃-alkyl"), carbon atoms and which is attached to the rest of the molecule by a single bond, including for example and in a non-limiting sense, methyl, ethyl, n-propyl, i-propyl, n-butyl, t-butyl, n-pentyl, etc.

The term "cycloalkyl" refers to a saturated or partially saturated mono- or polycyclic aliphatic group having between 3 and 8, preferably between 3 and 6 carbon atoms which is bound to the rest of the molecule by means of a single bond, including for example and in a non-limiting sense, cyclopropyl, cyclohexyl, cyclopentyl, etc.

The term "aryl" refers to an aromatic group having between 6 and 10, preferably between 6 and 8, comprising 1 or 2 aromatic nuclei, bound by means of a carbon-carbon bond or fused, including for example and in a non-limiting sense, phenyl, naphthyl, diphenyl, indenyl, etc. Preferably "aryl" refers to phenyl.

The term "arylalkyl" refers to an aryl group attached to the rest of the molecule through an alkyl group, e.g. C₆-C₁₀-aryl-C₁-C₂-alkyl.

The term "perfluoroalkyl" refers to an alkyl group wherein all possible positions are substituted by ―F.

The term "C₁₋₆ alkenyl" or "C₁-C₆-alkenyl" refers to a linear or branched hydrocarbon chain radical consisting of carbon and hydrogen atoms, containing at least one unsaturation, having between 1 and 6, preferably between 1 and 3 ("C₁₋₃ alkenyl" or or "C₁-C₃-alkenyl"), carbon atoms and which is attached to the rest of the molecule by a single bond, including for example and in a non-limiting sense, ethenyl, n-propenyl, etc.

"Heterocyclyl" refers to a stable 3- to 10-membered ring radical, which consists of carbon atoms and from one to five heteroatoms selected from the group consisting of nitrogen, oxygen, and sulphur and which can be partially or fully saturated. For the purposes of this invention, the heterocycle can be a monocyclyl, bicyclyl or tricyclyl ring system, which can include systems of fused rings; and the nitrogen, carbon or sulfur atoms in the heterocyclyl radical may be optionally oxidized; and the nitrogen atom may be optionally quaternized. Examples of such heterocycles include, but are not limited to, pyrrolidine, piperidine, piperazine, morpholine, tetrahydrofuran. According to one embodiment, the heterocyclyl group is a 5- or 6-membered ring. According to a further embodiment, the heterocyclyl group has between 6 and 10 carbon atoms (C₆-C₁₀).

"Heteroaryl" refers to a stable 5- or 6-membered aromatic ring, which consists of carbon atoms and from one to five heteroatoms selected from the group consisting of nitrogen, oxygen, and sulphur. For the purposes of this invention, the heteroaryl can be a monocyclyl, bicyclyl or tricyclyl ring system, which can include systems of fused rings; and the nitrogen, carbon or sulfur atoms in the heteroaryl radical may be optionally oxidized; and the nitrogen atom may be optionally quaternized. Examples of such heteroaryl include, but are not limited to, benzimidazole, benzothiazole, furan, pyrrole, pyridine, pyrimidine, isothiazole, imidazole, indole, purine, quinoline, thiadiazole. Preferably, "heteroaryl" refers to tiophene.

The term "halogen" or "halo" refers to bromo, chloro, iodo or fluoro.

As understood in this technical area, there can be a certain degree of substitution on the previously defined radicals. Thus, there can be substitution in any of the groups of the present invention. The references of the present document to substituted groups in the groups of the present invention indicate that the specified radical can be substituted in one or more available positions by one or more substituents. Said substituents include, for example and in a non limiting sense, C₁₋₆ alkyl, cycloalkyl, aryl, heterocyclyl, heteroaryl, halogen, cyano, nitro, trifluoromethyl, -N(Rₐ)(R_{b}), -OR_{c}, -SR_{d}, -C(O)Rₑ, -C(O)OR_{f}, -C(O)N(Rg)(Rₕ), -OC(O)R_{¡}; wherein Rₐ, R_{b}, R_{c}, R_{d}, Rₑ, R_{f}, Rg, Rₕ and Rᵢ are independently selected from hydrogen, C₁-C₆ alkyl, aryl, heterocyclyl, heteroaryl and trifluoromethyl.

According to an embodiment of the invention, A is -C(H)- and B is ―O-.

According to an embodiment of the invention, C is a phenyl group optionally substituted by one or more groups independently selected from ―F, -Cl, -Br, ―I, C₁-C₃ alkyl (e.g. methyl), C₁-C₃ alkenyl, C₁-C₃ perfluoroalkyl, -O-C₁-C₃-alkyl (e.g. methoxy), -O-C₁-C₃-alkenyl, -CN, C₆-C₁₀ aryl, C₇-C₁₀ arylalkyl, wherein the alkyl groups of said C₁-C₃ alkyl and said O-C₁-C₃-alkyl groups can be substituted by a ―O-C₆-C₁₀ heteroaryl group. According to a further embodiment, C is a phenyl group at least substituted with -F, -Cl, -Br or ―I, more particularly with ―F or ―Cl.

According to a further embodiment, C is a phenyl group of formula (VII) 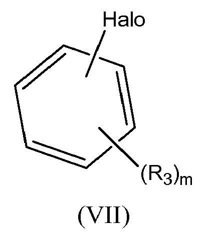 wherein Halo, R₃ and m are as defined above.

According to an embodiment of the invention, Ar is a phenyl group. According to an embodiment of the invention, Ar is selected from the group consisting of a furane, a thiophene and a phenyl group substituted by ―F, -Cl, -Br or ―I.

According to an embodiment of the invention, n is cero.

According to an embodiment of the invention, R₁ is hydrogen.

According to a further embodiment, R₁ is a C₁-C₆alkyl group substituted by - NH₂, -SH or ―OH.

According to a further embodiment, R₁ is a group of formula (VIII) 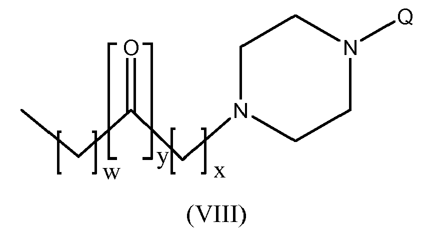 wherein x, y and w are as defined above, at least one of x, y or w being different from 0, and Q is a phenyl group optionally substituted by one or two groups selected from the group consisting of fluor, chloro, bromo, iodo and ―O-C₁-C₆ alkyl, or Q is 6-membered heteroaryl group having one of two nitrogen atoms in the ring. According to a more particular embodiment, Q is a phenyl ring substituted by one or two groups selected from the group consisting of fluor, chloro, bromo, iodo and methoxy. According to a even more particular embodiment, Q is a phenyl group substituted by one fluoro, one chloro or one methoxy. According to a even more particular embodiment embodiment, Q is p-fluorophenyl, m-chlorophenyl, p-chloropehnyl or o-methoxyphenyl.

According to a further embodiment, x, y and w are as defined above, at least one of x, y or w being different from 0, and Q is pyperazinyl or priridinyl, preferably, pyperazin-2-yl.

According to a further embodiment, the piperidine group is attached to the rest of the molecule through position 3. According to a further embodiment, the piperidine group is attached to the rest of the molecule through position 4.

According to another embodiment of the present invention, the compounds of formula (I) are preferably selected from the group consisting of:

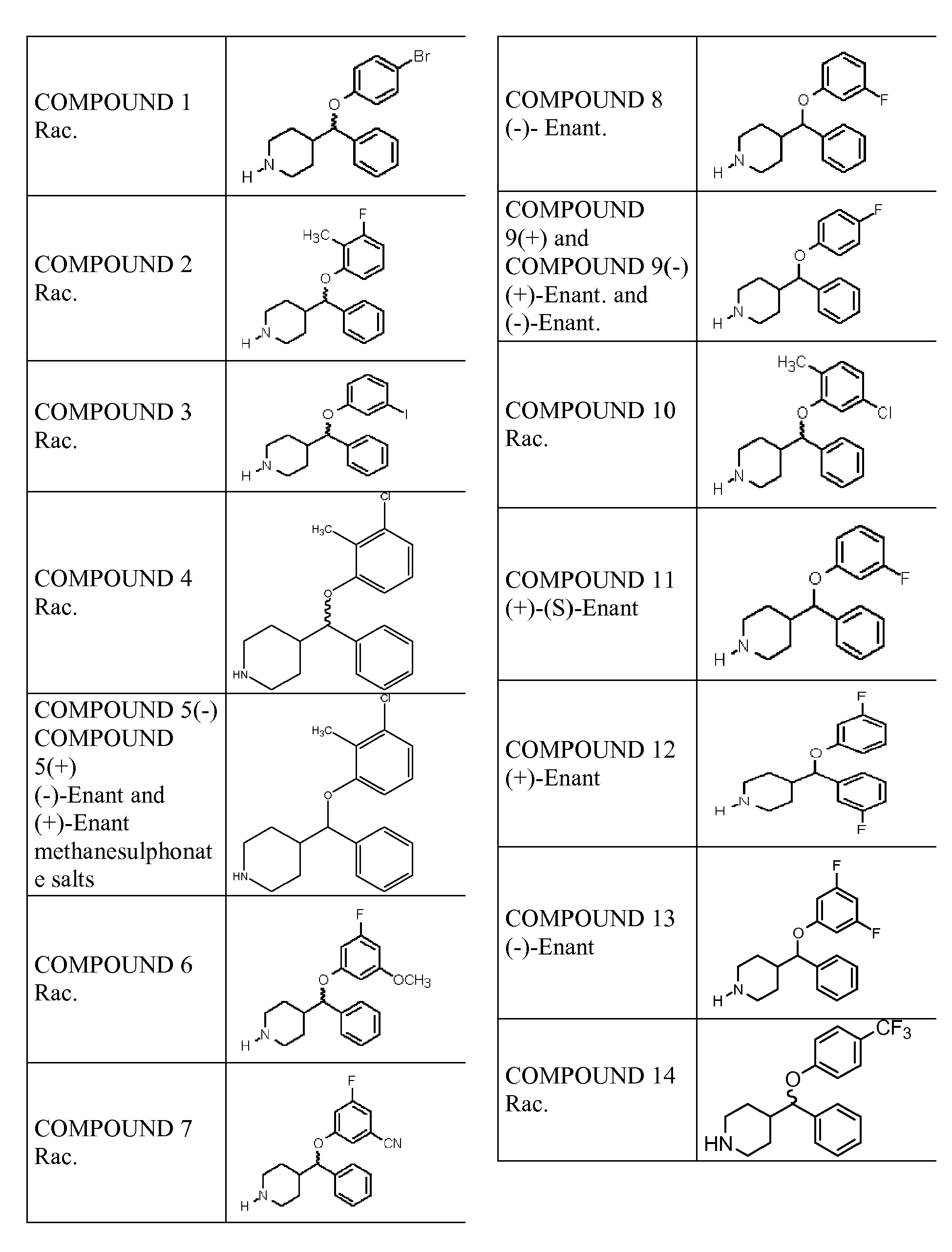

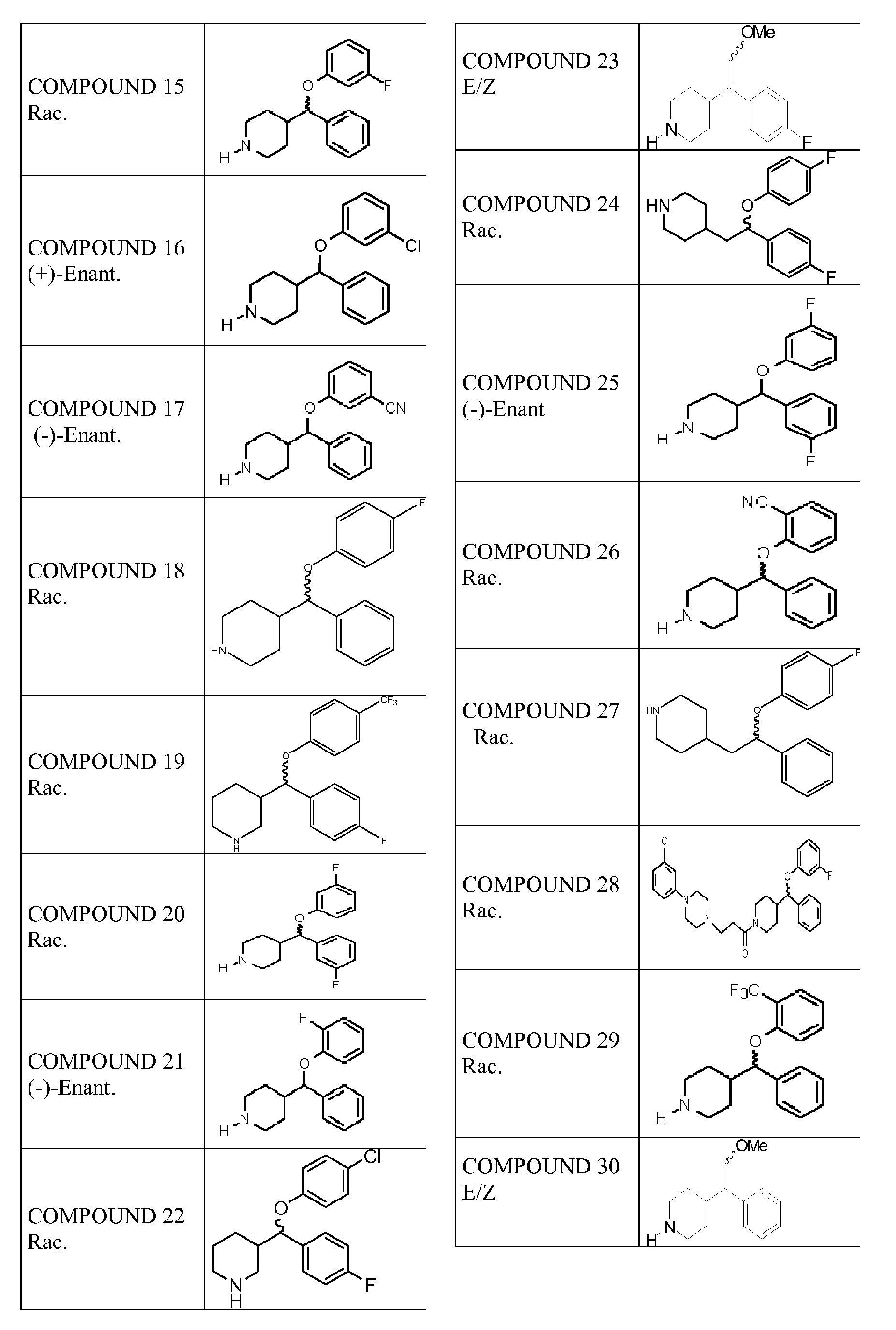

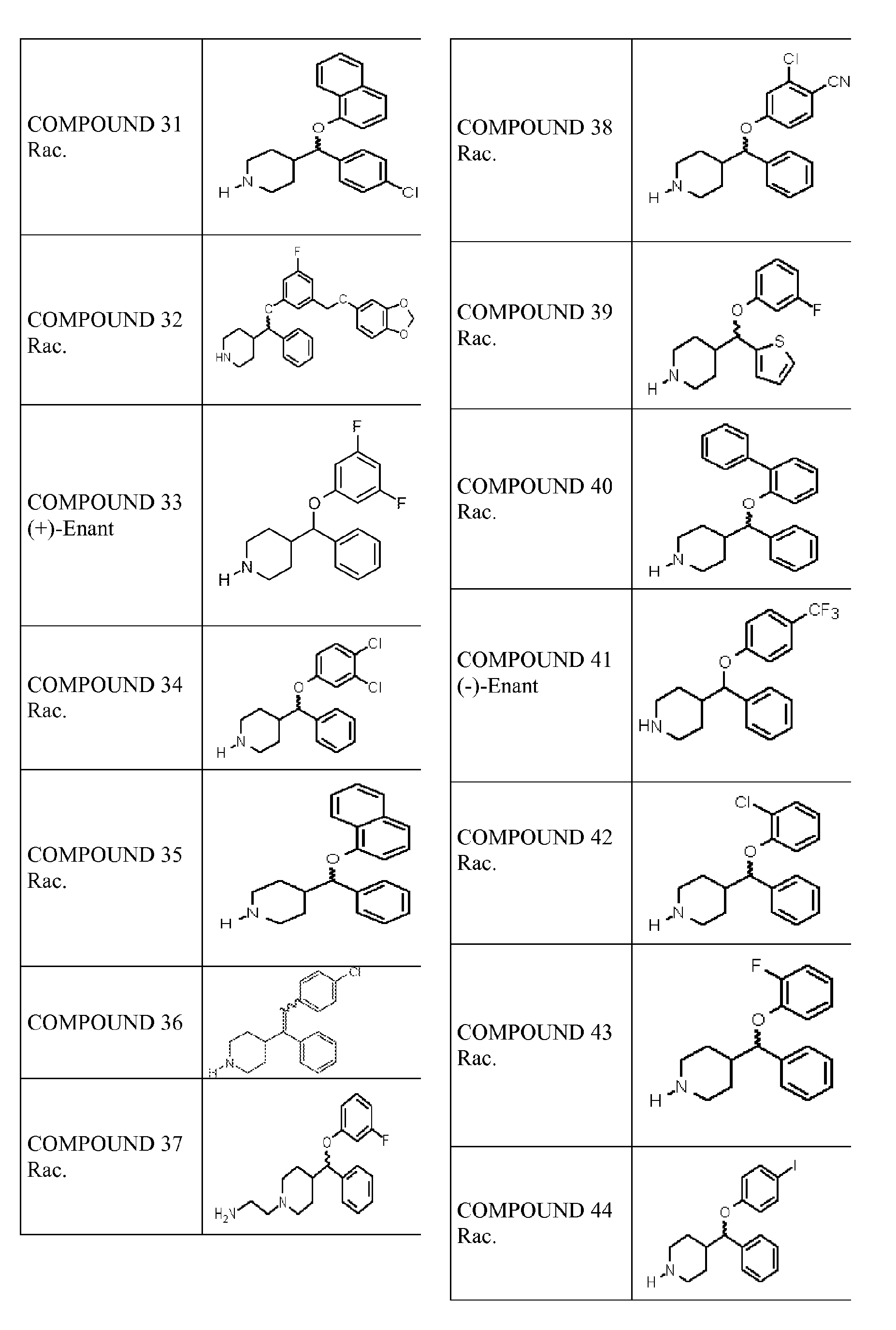

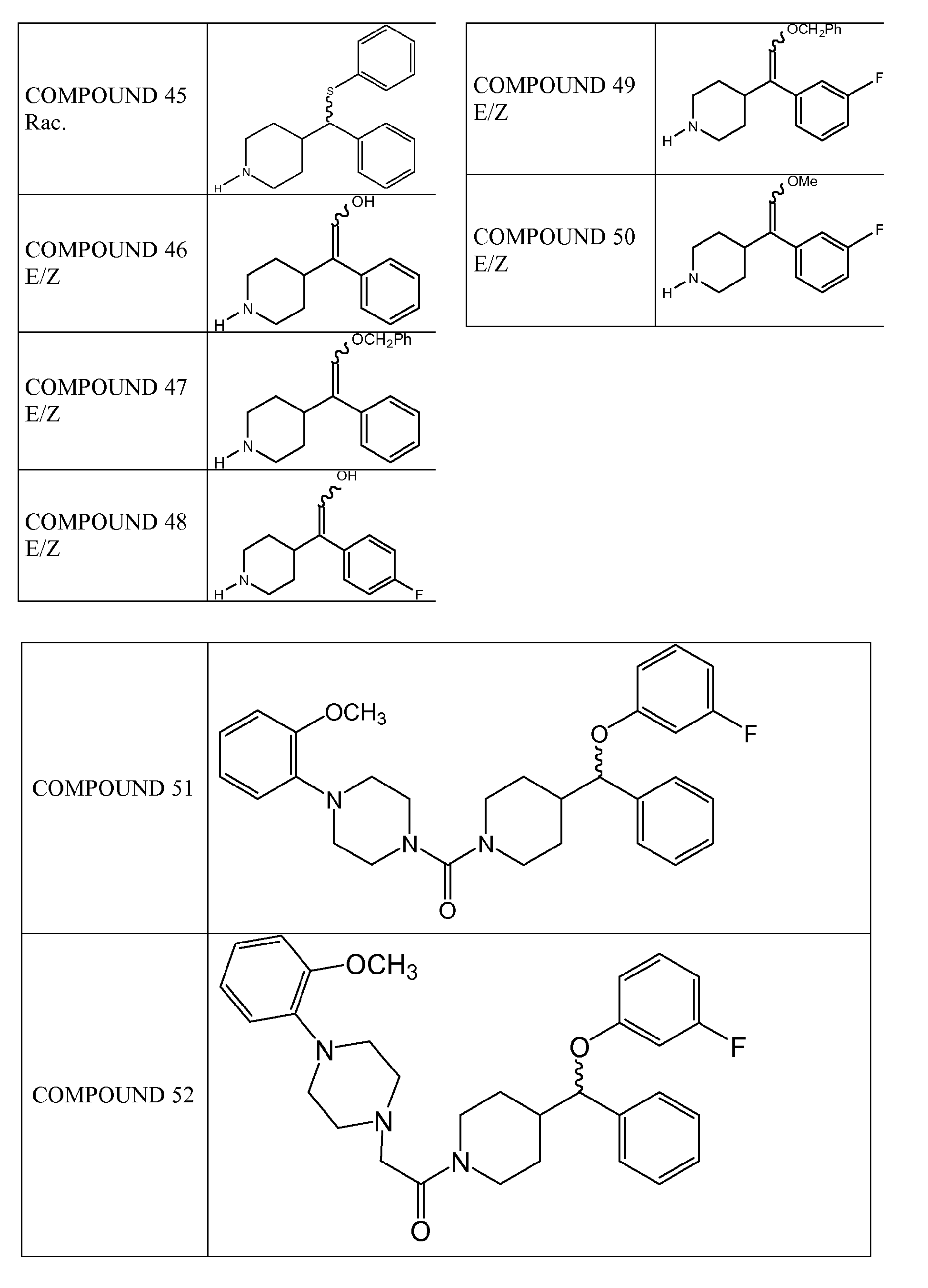

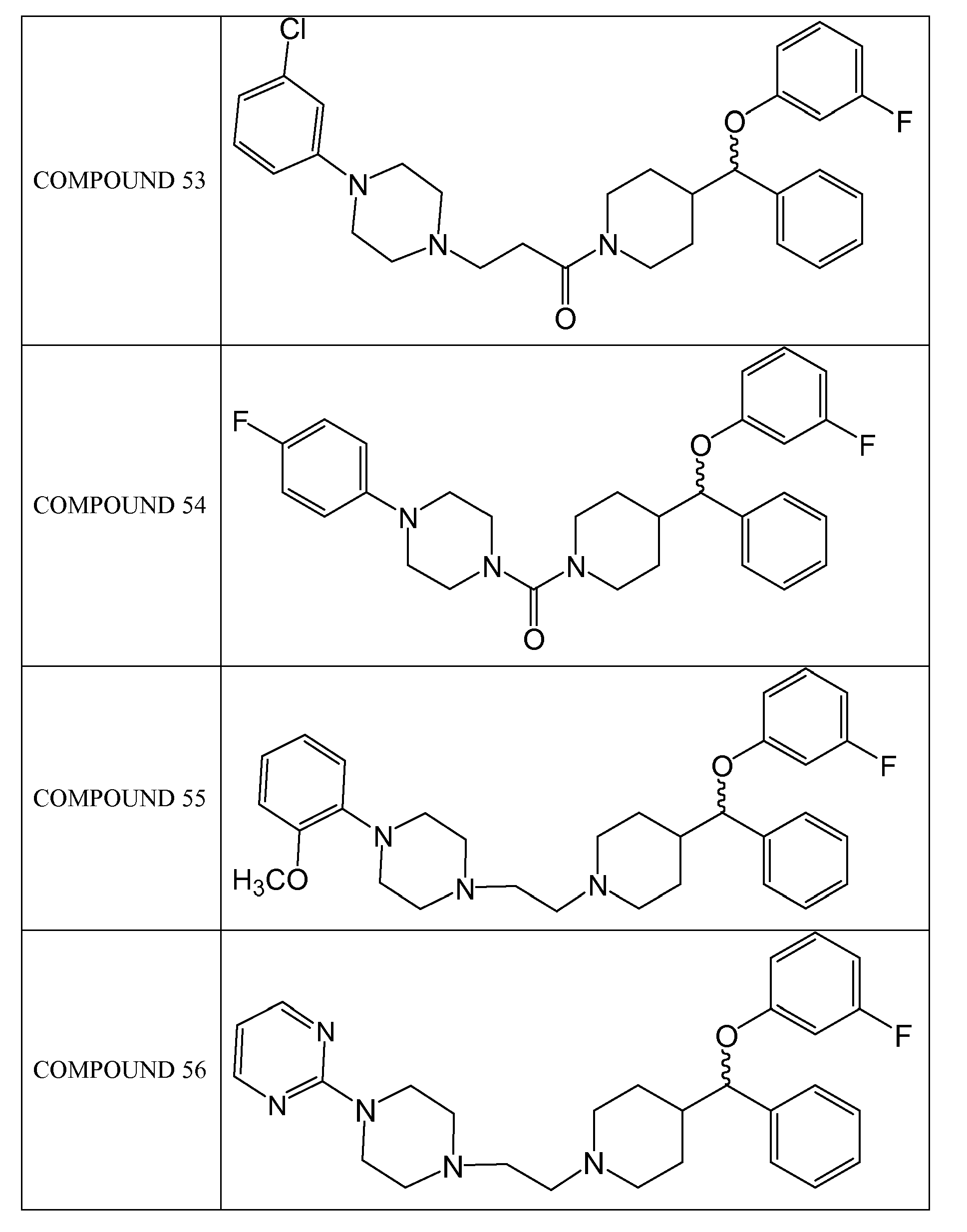

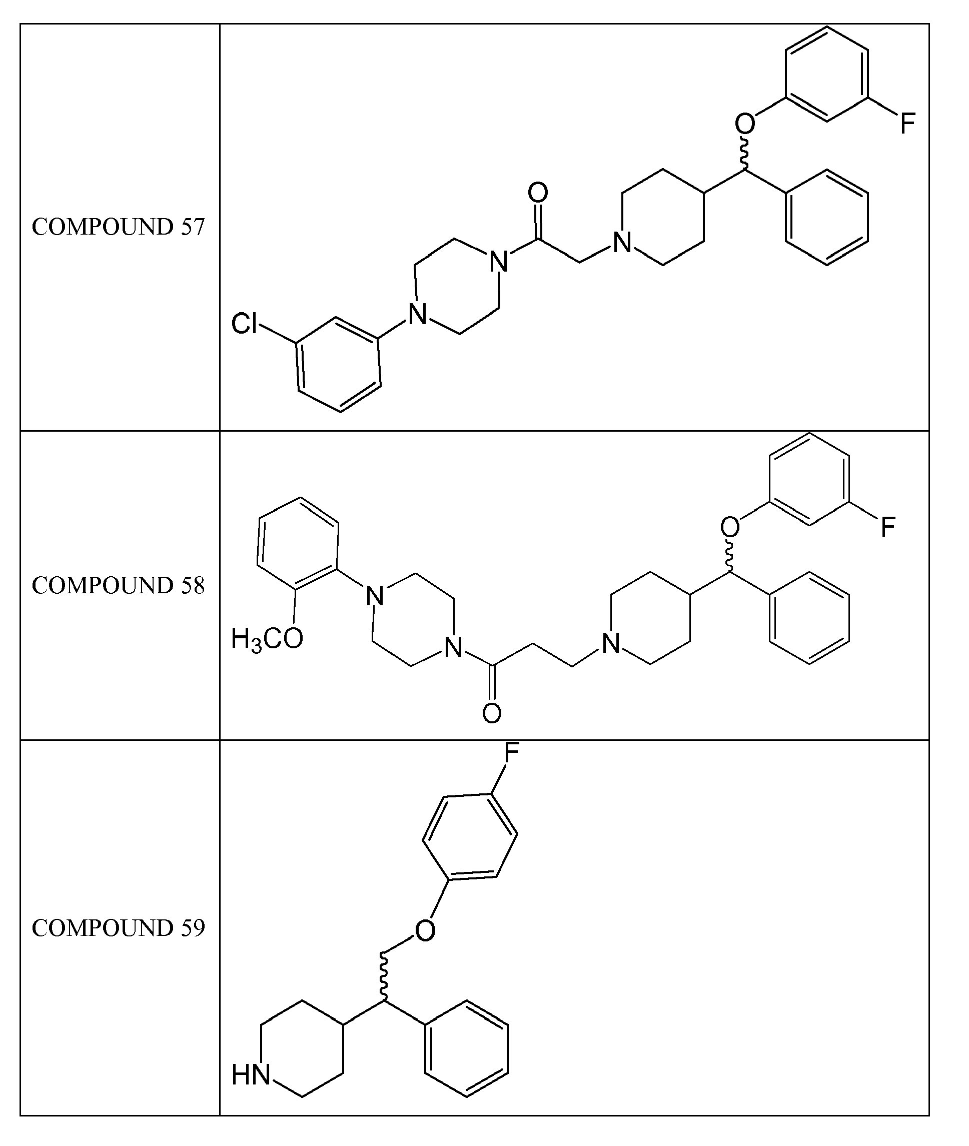

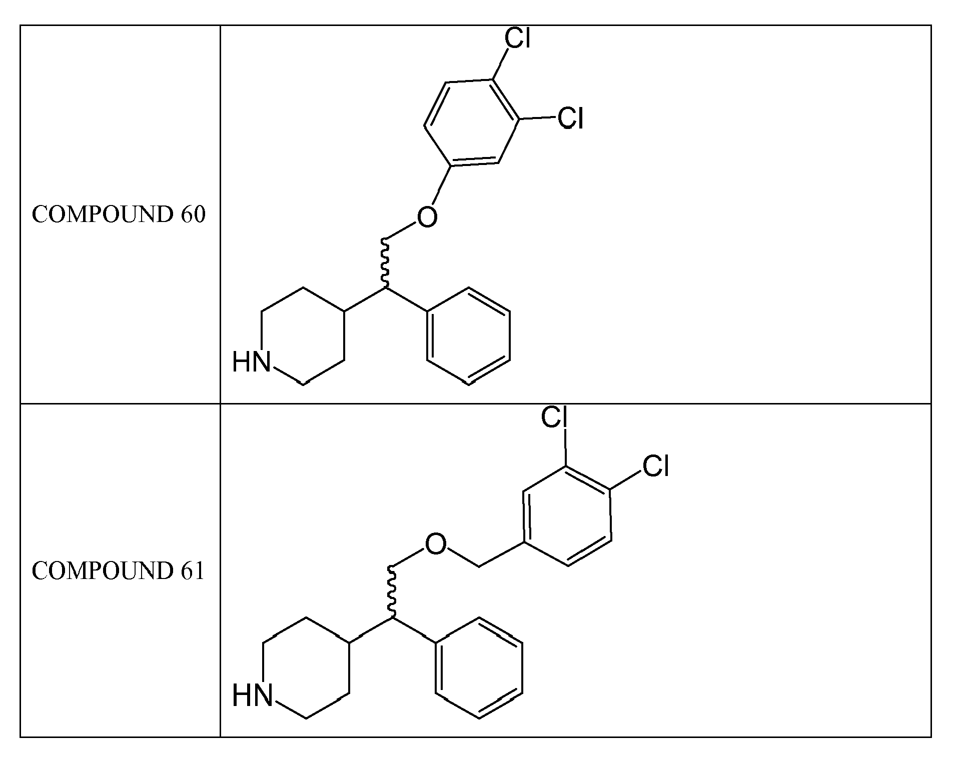

or a pharmaceutically acceptable salt, stereoisomer and/or solvate thereof.

A further embodiment of the invention is the compound COMPOUND 3 or a pharmaceutically acceptable salt, stereoisomer and/or solvate thereof.

A further embodiment of the invention is the compound COMPOUND 10 or a pharmaceutically acceptable salt, stereoisomer and/or solvate thereof.

A further embodiment of the invention is the compound COMPOUND 4 or a pharmaceutically acceptable salt, stereoisomer and/or solvate thereof.

A further embodiment of the invention is the compound COMPOUND 9(+) or a pharmaceutically acceptable salt, stereoisomer and/or solvate thereof.

A further embodiment of the invention is the compound COMPOUND 5(-) or a pharmaceutically acceptable salt, stereoisomer and/or solvate thereof.

A further embodiment of the invention is the compound COMPOUND 5(+) or a pharmaceutically acceptable salt, stereoisomer and/or solvate thereof.

A further embodiment of the invention is the compound COMPOUND 31 or a pharmaceutically acceptable salt, stereoisomer and/or solvate thereof.

A further embodiment of the invention is the compound COMPOUND 41 or a pharmaceutically acceptable salt, stereoisomer and/or solvate thereof.

A further embodiment of the invention is the compound COMPOUND 34 or a pharmaceutically acceptable salt, stereoisomer and/or solvate thereof.

A further embodiment of the invention is the compound COMPOUND 29 or a pharmaceutically acceptable salt, stereoisomer and/or solvate thereof.

A further embodiment of the invention is the compound COMPOUND 37 or a pharmaceutically acceptable salt, stereoisomer and/or solvate thereof.

A further embodiment of the invention is the compound COMPOUND 40 or a pharmaceutically acceptable salt, stereoisomer and/or solvate thereof.

A further embodiment of the invention is the compound COMPOUND 27 or a pharmaceutically acceptable salt, stereoisomer and/or solvate thereof.

A further embodiment of the invention is the compound COMPOUND 24 or a pharmaceutically acceptable salt, stereoisomer and/or solvate thereof.

A further embodiment of the invention is the compound COMPOUND 32 or a pharmaceutically acceptable salt, stereoisomer and/or solvate thereof.

A further embodiment of the invention is the compound COMPOUND 18 or a pharmaceutically acceptable salt, stereoisomer and/or solvate thereof.

A further embodiment of the invention is the compound COMPOUND 36 or a pharmaceutically acceptable salt, stereoisomer and/or solvate thereof.

A further embodiment of the invention is the compound COMPOUND 35 or a pharmaceutically acceptable salt, stereoisomer and/or solvate thereof.

A further embodiment of the invention is the compound COMPOUND 21 or a pharmaceutically acceptable salt, stereoisomer and/or solvate thereof.

According to an embodiment of the invention, R⁵ in a compound of formula (III) is methyl. According to an embodiment of the invention, R⁵ in a compound of formula (III) is hydrogen. According to an embodiment of the invention, R⁵ in a compound of formula (III) is benzyl.

According to an embodiment of the invention, R⁴ in a compound of formula (II) is a C₂-C₄-alkyl group optionally substituted by an amino group.

According to an embodiment of the invention, R₃ is hydrogen in compound of formula (III), (IV), or (V).

According to an embodiment of the invention, "J" is furan or thiophene in a compound of formula (V).

The compounds of formula (I) may be in the form of salts, preferably pharmaceutically acceptable salts, or in the form of solvates. When administered to the recipient, said salts or solvates can provide (directly or indirectly) a compound such as the one described herein. Nevertheless, it will be observed that pharmaceutically unacceptable salts are also within the scope of the invention because they can be useful for preparing pharmaceutically acceptable salts. Salts can be prepared by means of methods known in the state of the art. "Pharmaceutically acceptable" preferably relates to molecular entities and compositions which are physiologically tolerable and do not typically cause an allergic reaction or a similar unfavorable reaction, such as gastric disorders, dizziness and the like, when administered to a human or animal. The term "pharmaceutically acceptable" means that it is approved by a regulatory agency of a federal or state government or is included in the US pharmacopoeia or another generally recognized pharmacopoeia for use in animals, and more particularly in humans.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate. Preferably, the solvates are pharmaceutically acceptable solvates.

The preparation of salts and solvates can be carried out by methods known in the art. For instance, pharmaceutically acceptable salts of compounds provided herein are synthesized from the parent compound, which contains basic moieties, by conventional chemical methods. Generally, such salts are, for example, prepared by reacting the free base forms of these compounds with the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, the mono- and di-salts of hydrochloride, hydrobromide, hydroiodide, sulphate, nitrate, phosphate, and organic acid addition salts such as, for example, the mono- and di- salts of acetate, maleate, fumarate, citrate, oxalate, succinate, tartrate, malate, mandelate, methanesulphonate and p-toluenesulphonate. In a preferred embodiment, the salt is the dichlorhydrate salt or the hemisuphate salt or methanesulphonate.

One preferred pharmaceutically acceptable form is the crystalline form, including such form in a pharmaceutical composition. In the case of salts and solvates the additional ionic and solvent moieties must also be non-toxic. The compounds of the invention may present different polymorphic forms, it is intended that the invention encompasses all such forms.

An "stereoisomer" in the present patent application makes reference to compounds made up of the same atoms bonded by the same sequence of bonds but having different three-dimensional structures which are not interchangeable, including enantiomers and diastereomers (or diastereoisomers) of a given compound or formula.

The compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or a nitrogen by ¹⁵N-enriched nitrogen are within the scope of this invention.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

### Pharmaceutical compositions

A further aspect of the invention is a pharmaceutical composition comprising a compound of formula (II), (III), (IV), or (V) as defined above, or a derivative as defined above, or a pharmaceutically acceptable salt, stereoisomer and/or solvate thereof, and at least one pharmaceutically acceptable carrier.

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 21st Edition, 2005. Preferably, the carriers of the invention are approved by a regulatory agency of the Federal or a state government or listed in the U.S. Pharmacopeia or other generally recognized pharmacopeia for use in animals, and more particularly in humans.

The carriers and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993 or "Handbook of Pharmaceutical Excipients", Rowe C. R.; Paul J. S.; Marian E. Q., sixth Edition.

Examples of pharmaceutical compositions include any solid (tablets, pills, capsules, granules etc.) or liquid (solutions, suspensions or emulsions) compositions for oral, topical or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

The compounds or compositions of the present invention may be administered by any suitable method, such as intravenous infusion, oral preparations, and intraperitoneal and intravenous administration. Oral administration is preferred because of the convenience for the patient and the chronic character of many of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.01 to 1000 mg/kg/day.

### EXAMPLES

The compounds of the invention, including the compounds of formula (II), (III), (IV), or (V), can be prepared following the general methods described in N° 9802420, EP1002794 Al, US 6,518,284, or PCT/EP2007/053582.

Also the compounds of formula (V) or related compounds can be prepared according to the Scheme 1 below by treatment of the corresponding ketone with Me₃S(O)I or MeOCH₂PPh₃ (P.J.Gilligan et al J. Med. Chem. 1992, 35, 23, 4349) followed by reduction. The resulting alcohol may be alkylated with the corresponding benzyl or phenyl derivative.

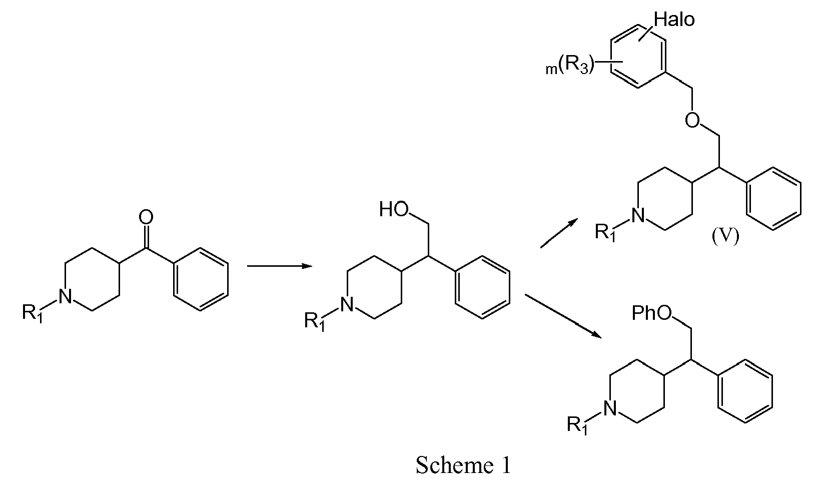

Further representative examples are given below:

### Example 1

### Preparation of (+/-)-4-[(Naphthalen-1-oxy)(4-chlorophenyl)methyl]piperidine hemisulphate COMPOUND 31

The following procedure is similar to the preparation of compound 15au described in J. Med. Chem. 2003, Vol 46, 25, 5512-5532.

Compound 14b of J Med Chem 2003, vol 46, 25, 5512-5532 (1.6 g, 4.8 mmol) was added portionwise to a stirred suspension of hexane-washed NaH (0.2 g, of a 60% oil dispersion) in 10 mL of anhydrous DMSO. The reaction was stirred at room temperature for 30 min, potassium benzoate (0.7 g) added, and stirring continued for 30 min. naphthalene (5.9 mmol) was added, the reaction mixture heated at 85 °C for 15 h and then cooled to room temperature. A mixture of water and brine was added and the oil extracted with diethyl ether. The organic layer was concentrated in vacuo, stirred and refluxe for 1.5 h with methanol (35 Ml) and 10% aqueous HCl solution (30 mL). After removing the solvent, the residue was partitioned between 10% aqueous HCl solution and CHCl₃. The aqueous acidic solution was treated with 5% aqueous NaOH solution until pH>9, the oil that separates was extracted with CHCl₃ dried over anhydrous Na₂SO₄, filtered and the solvent removed under reduced pressure to yield an amber oil. The crude product was stirred for 30 min with a solution of H₂SO₄ (0.4 mL) in water (20 mL). The slightly brown solid formed was filtered and washed with water to afford 1.2 g (Yield: 67 %) of the title compound.

### Example 2

### Preparation of (+/-)-4-[3-Fluoro-5-[(3,4-methylenedioxy)phenoxy]methyl]phenoxy] methyl]piperidine hydrochloride COMPOUND 32

To a solution of 3,4-(Methylene)dioxyphenol (Sesamol or 5-Benzodioxolol) (3.4 g, 25 mmol) in acetonitrile (50 mL) was added anhydrous K₂CO₃ (4.2 g) and then 3,5-Difluorobenzyl bromide (4.2 g, 25.0 mmol). The mixture was refluxed for 6 h, cooled to room temperature and the solvent removed under reduced pressure. A 5% aqueous NaOH solution was added to the residue and the mixture extracted with CH₂Cl₂. The organic layer was dried over anhydrous Na₂SO₄, filtered and concentrated in vacuo to give 1-[(3,4-methylenedioxy)phenoxy]methyl-3,5-difluorobenzene as a slightly yellow solid (mp 53-56 °C, yield: 78 %).

Compound 14j of J. Med. Chem. 2003, vol 46, 25, 5512-5532 (2.0 g, 10.5 mmol) was added portionwise to a stirred suspension of hexane-washed NaH (0.6 g, of a 60% oil dispersion) in 20 mL of anhydrous DMSO. The reaction was stirred at room temperature for 1 h, potassium benzoate (0.2 g) added, and stirring continued for 30 min. A solution of 1-[(3,4-methylenedioxy)phenoxy]methyl-3,5-difluorobenzene (3.0 g, 11.4 mmol) in 5 mL of anhydrous DMSO was added, the reaction mixture stirred at room temperature for 20 h, treated with water and 10 % aqueous HCl solution and extracted with CHCl₃. The organic layer was washed with 10 % aqueous NaOH solution, dried over anhydrous Na₂SO₄, filtered and concentrated in vacuo to give an amber oil (2.9g, Yield: 70 %) which was stirred for 30 min with a saturated solution of HCl in diethyl ether to afford the title compound as a slightly brown solid (mp 55 °C (d)).

### Example 3

### Preparation of (+/-)-4-[(3-Fluorophenoxy)(2-thiophenyl)methyl]piperidine oxalate COMPOUND 39

To 1-acetyl-piperidine-4-carboxylic acid (4.3 g, 26.0 mmol) was added a mixture of 85% phosphoric acid (2 mL) and thiophene (4 mL). The reaction mixture was treated dropwise with trifluoroacetic anhydride (15 mL, an exothermic reaction was observed) and heated to 80-90 °C for 4h. The reaction was cooled to room temperature and partitioned between water and CHCl₃. The organic layer was washed with 10% aqueous NaOH solution, dried over anhydrous Na₂SO₄, filtered and the solvent removed under reduced pressure to give (1-acetylpiperidin-4-yl)(2-thiophenyl)methanone as a slightly yellow solid (6.2 g, yield: 98 %, mp 112-116 °C).

A mixture of (1-acetylpiperidin-4-yl)(2-thiophenyl)methanone (6.1 g, 26.0 mmol) and methanol (50 mL) was treated dropwise with a solution of NaBH₄ (0.7 g) in water (5 mL). The reaction mixture was heated at 60 °C for 3 h, cooled to room temperature, treated with 350 mL of water and extracted with CHCl₃. The organic layer was dried over anhydrous Na₂SO₄, filtered and concentrated under reduced pressure to give (+/-)-(1-acetylpiperidin-4-yl)(2-thiophenyl)methanol as an slightly yellow oil (5.6 g, yield: 93%).

(+/-)-(1-acetylpiperidin-4-yl)(2-thiophenyl)methanol (3.5 g, 17.8 mmol) was added to a stirred suspension of hexane-washed NaH (1.1 g, of a 60% oil dispersion) in 30 mL of anhydrous DMSO. The reaction was stirred at room temperature for 30 min, potassium benzoate (0.3 g) added and stirring continued for 30 min. 1,3-Difluorobenzene (2.2 mL) was added and the reaction mixture stirred at room temperature for 20 h. Then, the reaction mixture was treated with water and 10 % aqueous HCl solution and extracted with CHCl₃. The organic layer was washed with 5 % aqueous NaOH solution, dried over anhydrous Na₂SO₄, filtered and concentrated in vacuo to give an amber oil (4.0 g, Yield: 77 %). The crude product (2.0 g) was dissolved in 5 mL of ethanol and oxalic acid (0.9 g, 6.8 mmol) added. The obtained solution was concentrated in vacuo to give a slightly yellow solid that was crystallized in dioxane (40 mL) to afford colorless crystals of the title compound (2.2 g, yield: 85 %, mp 142-143 °C).

### Example 4

### Preparation of (+/-)-3-[4-(3-chlorophenyl)piperazin-1-yl ]-1-[4-[(3-fluorophenoxy) pheylmetyl]piperidin-1-yl]propanone hydrochloride COMPOUND 28

A solution of compound 15j of J. Med. Chem. 2003, vol 46, 25, 5512-5532 (3.0 g, 10.5 mmol) in dichloromethane (10 mL) was cooled to 0-5 °C and a solution of 3-chloropropionyl chloride (1.5 mL, 15 mmol) in dichloromethane (4 mL) was added dropwise. The reaction mixture was stirred at room temperature for 20 h and then washed with 5 % aqueous NaOH solution. The organic layer was dried over anhydrous Na₂SO₄, filtered and concentrated under reduced pressure to afford a slightly yellow oil (3.65 g). A sample of the crude product (1.9 g) was purified by flash chromatography on silica gel (EtOAc:hexane 6/4) to give (+/-)- 3-chloro-1-[4-[(3-fluorophenoxy)phenylmethyl]piperidin-1 -yl]propanone.

To a solution of (+/-)- 3-chloro-1-[4-[(3-fluorophenoxy)phenylmethyl]piperidin-1-yl]propanone (1.7 g, 4.6 mmol) in acetonitrile (35 mL) were added anhydrous K₂CO₃ (0.9 g), KI (10 mg) and 1-(3-chlorophenyl)piperazine (1.0 g, 5.2 mmol). The mixture was heated at 85 °C for 24 h, cooled to room temperature and the solvent removed under reduced pressure. Water was added to the residue and the mixture extracted with CHCl₃. The organic layer was dried over anhydrous Na₂SO₄, filtered and concentrated in vacuo to give a residue that was purified by flash chromatography on silica gel (CHCl₃/Methanol/Isopropilamine 95/5/5) to afford (+/-)-3-[4-(3-chlorophenyl)piperazin-1-yl]-1-[4-[(3-fluorophenoxy)phenylmetyl]piperidin-1-yl]propanone as an oil which was treated with a saturated solution of HCl in diethyl ether to give the title compound as a slightly brown solid (0.75 g, mp 150-152 °C).

### Example 5

### Preparation of (+/-)-4-[(3-fluorophenoxy)phenylmetyl]piperidin-1-yl]ethylamine oxalate COMPOUND 37

A mixture of compound 15j of J. Med. Chem. 2003, vol 46, 25, 5512-5532 (2.9 g, 10.3 mmol), anhydrous K₂CO₃ (6 g), 2-(2-bromoethyl)-1,3(2*H*)-isoindoldione (3.2 g, 13 mmol) and dioxane (40 mL) was heated at 115 °C for 24 h. The reaction mixture was cooled to room temperature and the solvent removed under reduced pressure. Water was added to the residue and the pH adjusted to 7.5 with a 3.5 % aqueous HCl solution. The mixture was extracted with CHCl₃ and the organic layer was dried over anhydrous Na₂SO₄, filtered and concentrated in vacuo to give a residue which was purified by flash chromatography on silica gel (EtOAc/hexane 8/2) to afford (+/-)-2-[2-[4-[(3-fluorophenoxy)phenylmethyl]piperidin-1-yl]ethyl]-1*H*-isoindol-1,3(2*H*)-dione as an orange oil (2.8 g, yield: 60 %).

To a solution of (+/-)-2-[2-[4-[(3-fluorophenoxy)phenylmethyl]piperidin-1-yl]ethyl]-1*H*-isoindol-1,3(2*H*)-dione (2.5 g) in 70 mL of ethanol was added hydrazine hydrate (98 %, 2 mL) and a white solid is observed. After 20 h at room temperature the white solid was filtered and washed with 5 % aqueous NaOH solution. The filtrate was concentrated under reduced pressure to give an slightly yellow oil. A sample of the crude product (0.8 g) was dissolved in 5 mL of ethanol and oxalic acid (0.3 g, 2.4 mmol) was added. A slightly brown solid was obtained, filtered and washed with diethyl ether to give 0.9 g of the title compound (mp 108 °C (d)).

### Example 6

### Preparation of ( Z )-4-[(1-phenyl)-2-(4-chlorophenyl)ethen-1-yl]piperidine COMPOUND 36

To a mixture of NaHCO₃ (173 g) and water (1.5 L) was added compound 11a of J. Med. Chem. 2003, vol 46, 25, 5512-5532 (119.3 g, 0.63 mol). The reaction was stirred at room temperature for 15 min and then (Boc)₂O was added. After stirring for 15 h at room temperature a white solid was filtered and washed with water to affor 164 g (Yield: 90 %, mp 91-93 °C) of 4-benzoyl-1-[(1,1-dimethylethyloxy)carbonyl]piperidine. A suspension of Mg turnings (1.0 g) in anhydrous diethyl ether (40 mL) was prepared and treated with 1/3 of a solution of 4-chlorobenzyl chloride (4.6 g, 27.0 mmol) in anhydrous diethyl ether (40 mL) and an iodine crystal. The mixture was heated until a smooth reflux was observed and the color disappeared. The rest of the solution of 4-chlorobenzyl chloride was added. The reflux was continued for 3.5 h and the reaction mixture cooled to room temperature. A solution of 4-benzoyl-1-[(1,1-dimethylethyloxy)carbonyl]piperidine (6.4 g, 22.4 mmol) in anhydrous diethyl ether (50 mL) was added dropwise and the reaction refluxed for 3h. A saturated aqueous NH₄Cl solution (50 mL) was added, the diethyl ether evaporated and the mixture extracted with CHCl₃. The organic layer was dried over anhydrous Na₂SO₄, filtered and concentrated in vacuo to give to give a solid which was washed with hexane. This solid was dissolved in chloroform (100 mL), treated with trifluoroacetic acid (8 mL) and refluxed for 15 h. The reaction mixture was cooled to room temperature, treated with 10 % aqueous NaOH solution and extracted with chloroform. The organic layer was dried over anhydrous Na₂SO₄, filtered and evaporated under reduced pressure to afford a mixture E/Z isomers. Purification by flash chromatography on silica gel (Diethyl ether:Isopropilamine 10/0.5) gave 1.6 g of the Z isomer of the title compound (Yield: 22 %, mp 121-124 °C).

### Example 7

### Preparation of (E,Z)-4-(1-phenyl-2-mehoxyvinyl)piperidine hydrochloride COMPOUND 30

To a mixture of compound 11a of J. Med. Chem. 2003, vol 46, 25, 5512-5532 (1.0 g, 5.3 mmol), water (20 mL) and sodium acetate (4 g) was added methoxyamine hydrochloride (3.0 g). The reaction mixture was heated at 80 °C for 3 h, cooled to room temperature, treated with 10 % aqueous KOH solution until pH > 9 and extracted with chloroform. The organic layer was dried over anhydrous Na₂SO₄, filtered and evaporated under reduced pressure to afford an oil which was treated with saturated solution of HCl in diethyl ether yielding a mixture E/Z of isomers of the title compound as a white solid (1.1 g, mp 202-204 °C).

### Example 8

### Preparation of (E,Z)-4-[1-(4-fluorophenyl)-2-methoxyvinyl]piperidine hydrochloride COMPOUND 23

To a mixture of compound 11c of J. Med. Chem. 2003, vol 46, 25, 5512-5532) (0.5 g), water (10 mL) and sodium acetate (4 g) was added methoxyamine hydrochloride (2.0 g). The reaction mixture was heated at 80 °C for 2 h, cooled to room temperature, treated with 10 % aqueous NaOH solution until pH > 9 and extracted with chloroform. The organic layer was dried over anhydrous Na₂SO₄, filtered and evaporated under reduced pressure to afford an oil which was treated with saturated solution of HCl in diethyl ether yielding a mixture E/Z of isomers of the title compound as a white solid (0.6 g, mp 201-206 °C).

### Example 9

### Preparation of (+/-)-4-[2-(4-Fluorophenoxy)-1-phenylethyl]piperidine COMPOUND 59

A solution of compound 2-phenyl-2-piperidin-4-ylethanol (2.6 g, 10.5 mmol) in THF (50 mL) was treated with triphenylphosphine (2.7 g, 10.5 mmol) and 4-fluorophenol (1.3 g, 11.9 mmol). Then, a solution of DEAD (1.7 mL) in THF (10 mL) was added dropwise. The reaction mixture was stirred at room temperature for 15 h and the solvent removed under reduced pressure. A 5 % aqueous NaOH solution was added to the residue and the mixture extracted with CH₂Cl₂. The organic layer was dried over anhydrous Na₂SO₄, filtered and concentrated in vacuo to give a residue that was treated at reflux with methanol (20 mL) and a 10 % aqueous HCl solution (60 mL) for 8 h. The reaction mixtures was allowed to cool to room temperature, treated with water (100 mL) and extracted with CH₂Cl₂. The organic layer was washed with 5 % aqueous NaOH solution, dried over anhydrous Na₂SO₄, filtered and concentrated in vacuo to give a residue which was purified by flash chromatography on silica gel (Chloroform/Isopropylamine 9/0.2) to give (+/-)-4-[2-(4-Fluorophenoxy)-1-phenylethyl]piperidine as a yellow oil (2.0 g, yield: 63 %).

### BIOLOGICAL ASSAYS

### Primary cell cultures

a) - Mouse cortex neurons: brains were obtained from 16-18-day old embryos. The two hemispheres, olfactory bulbs and brain stem were separated. The meninges were removed and the cut tissue was transferred to a 50 ml flask. 10 ml HBSS without Ca without Mg (without phenol red) were added. Two further washings with 3 ml HBSS were performed and digestion was performed with Trypsin-DNase at 37°C. The suspension was broken up by passing the material through glass pipettes with different thicknesses. The cells were cut and cultured in Dulbecco's Modified Eagle's medium (DMEM)+10% FCS. After 3 hours the final medium was placed in Neurobasal medium + B27 Supplement + KCl + glutamine + antibiotics.
b) CGN: Cerebellar granule neurons derived from wild-type or transgenic mice were obtained from 6-day old mice. The basic process was similar to the previous one: obtaining and breaking up the tissue was similar and culturing and maintaining in final media was identical. The cerebellum was broken up by treatment with trypsin-EDTA for about 15-20 min and subsequently cultured in plates treated with Poly-L-Lysine (50 micrograms/ml). The neurons were maintained in a serum-free medium, Neurobasal medium + B27 Supplement + KCl + glutamine + antibiotics.

### Toxicity and cell viability

The cell viability of different strains was studied in the presence of the compounds of the invention. Thus, the cell viability was studied:
- In primary cultures of CGN with compounds COMPOUND 3, COMPOUND 10, COMPOUND 4, COMPOUND 9(+), COMPOUND 41, COMPOUND 34, COMPOUND 35, COMPOUND 29, COMPOUND 36; COMPOUND 31 and COMPOUND 37, the last two being somewhat toxic only at certain concentrations.
- In cultures of cell line CHO-APP-PS1 with compounds COMPOUND 3, COMPOUND 10 and COMPOUND 4, none of them giving rise to statistically relevant toxicity levels.
- In the primary culture of neurons derived from Tg-APP-PS1 mice with compounds COMPOUND 3, COMPOUND 10, COMPOUND 4, COMPOUND 5(+) and COMPOUND 5(-), the latter showing toxicity only at certain concentrations.

In the toxicity assays, the cells (whether neurons or established lines) were seeded in 24-well plates and exposed to different concentrations of the drug in Dulbecco's Modified Eagle's medium (DMEM)+10% FCS or in the Neurobasal medium + B27 Supplement + KC1 + glutamine + antibiotics when primary neurons were used. The treatments were stopped after 24 or 48 hours by fixing the cells with 4% paraformaldehyde for 30 minutes. The fixed cells were stained with 0.2% Coomassie Brilliant blue R-250 in 10% acetic acid/40% methanol for 1 hour at ambient temperature. The stained cells were washed thoroughly with distilled water, and once dried the incorporated die was extracted with 0.1 N NaOH in 50% methanol. Then it was acidified with 10% trichloroacetic acid and the number of cells was measured in an Elisa reader, with absorbance at 595 nm. DMSO (10 µl/well) was used as a control.

### Measurement of the release of the amyloid peptide

The line Chinese hamster ovary cell (CHO) stably transfected with APP-PS1 was used; it was maintained in Dulbecco's Modified Eagle's medium (DMEM) with 10% inactivated bovine fetal serum, Glutamine (2 mM) and a mixture of antibiotics (Penicillin/Streptomycin) and G-418 antibiotic (200 µg/ml). The cells were maintained in continuous growth and the medium was replaced with a new medium before applying the drugs to be assayed. After that the culture media in the different treatments were collected, always taking the corresponding solvent controls. The medium was collected for 6 hours, and then it was diluted 5 times and analyzed in duplicate using a Biosource ELISA kit (h 1-40 ßAmyloid - Ref # KHB3482).

For the primary cultures of cerebellum derived from APP-PS1 transgenic mice, the process was similar except that the culture medium corresponded to this kind of culture (NB-B27) and the medium was collected 24 hours after the compound was added or not. The final data was obtained from the interpolation in the ßAmyloid standard curve of the kit to express the final datum in concentration of ßAmyloid secreted per milliliter.

### Example 10: Ability to modify levels of tau phosphorylation in primary culture of cerebellar granule neurons (CGN) and/or brain cortex derived from mice (wild strain).

Based on the study of the ability to modify the level of tau phosphorylation, it can be concluded that compounds COMPOUND 3, COMPOUND 10, COMPOUND 4, and COMPOUND 9(+) modify tau phosphorylation. Compounds COMPOUND 5(-), COMPOUND 5(+), COMPOUND 31, COMPOUND 41, COMPOUND 34, COMPOUND 29, COMPOUND 37, COMPOUND 40, COMPOUND 27, COMPOUND 24, COMPOUND 32, COMPOUND 18, COMPOUND 36, COMPOUND 35, and COMPOUND 21 also modify tau phosphorylation at 5 and/or 10 micromolar.

The data was obtained in relation to the mean of 3 independent experiments of the relative level of tau-1 in soluble cell extracts obtained from cerebellar granule or brain cortex neurons treated with the indicated compounds. The results are shown in Figure 1. The ability to modify the level of tau phosphorylation in cortex neurons or cerebellar granule of rats was analyzed through Western Blot using specific antibodies in a series of fosfo-epitopes (Tau 1, PHF1) after 1, 2 and 3 incubation hours. Actine was used as reference (indicator of the total mass).

### Example 11: Ability to modify the secretion levels of the amyloid peptide (β1-40) of transgenic cell line CHO-APP-PS1.

The measurement was obtained in ng/ml secreted in the culture medium and then it was normalized in relation to the controls, which is the solvent, which in each case was considered 100%. The data obtained in 5-6 experiments is indicated in duplicate per assay except in the case of compound COMPOUND 9(+) in which it was n= 3. The results are shown in Figure 2 (COMPOUND 3), Figure 3 (COMPOUND 10), Figure 4 (COMPOUND 9(+)), and Figure 5 (COMPOUND 4).

### Example 12: Ability to modify the secretion levels of the peptide β1-40 in primary culture of cerebellar neurons derived from Tg-APP-PS1 transgenic mice.

The compounds were initially assayed at two times, 24 and 48 hours, and at two concentrations, 2 and 5 µM. The data represents the mean of three different experiments performed at 24 hours. The results are shown in Figure 6 (COMPOUND 3, COMPOUND 10 and COMPOUND 4), Figure 7 (COMPOUND 10), Figure 8 (COMPOUND 31 and COMPOUND 41), Figure 9 (COMPOUND 34, COMPOUND 29 and COMPOUND 37), Figure 10 (COMPOUND 2, COMPOUND 36 and COMPOUND 35) and Figure 11 (COMPOUND 5(+) and COMPOUND 5(+)).

## Claims

1. A compound of formula (I): 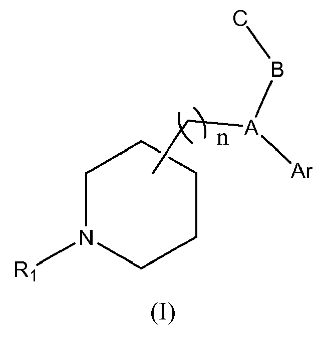 wherein:
R₁ is selected from the group consisting of hydrogen or -(CH₂)_{w}-(C=O)_{y}―(CH₂)ₓ-R₂, wherein R₂ is a C₁-C₆ alkyl group optionally substituted by -NH₂, -SH, -OH or C₃-C₈ cycloalkyl, or R₂ is a heterocyclyl group optionally substituted by a substituted or unsubstituted phenyl, or by a substituted or unsubstituted 5- or 6-membered heteroaryl group; w is an integer selected from 0, 1, 2 or 3; x is an integer selected from 0, 1, 2 or 3; and y is 0 or 1;
Ar is an optionally substituted phenyl or an optionally substituted 5- or 6-membered heteroaryl group;
when A is ―C(H)-; B is ―(CH₂)-O-, ―(CH₂)-S-, ―O- or ―S-; and C is an optionally substituted phenyl, an optionally substituted benyl or an optionally substituted naftyl group; or
when A is ―C(=)-; B is =C(H)-; and C is selected from the group consisting of ―OH, ―O(C₁-C₃-alkyl), and -O(C₇-C₁₀-arylalkyl), or is an optionally substituted phenyl or an optionally substituted naphtyl group; and
n is an integer selected from 0, 1 or 2;
or a pharmaceutically acceptable salt, stereoisomer and/or solvate thereof for use in a method to treat or ameliorate amyloid or tau pathologies, or symptoms thereof.

2. A compound according to claim 1 wherein A is -C(H)- and B is ―O-.

3. A compound according to any of the previous claims wherein C is a phenyl group optionally substituted by one or more groups independently selected from ―F, -Cl, - Br, ―I, C₁-C₃ alkyl, C₁-C₃ alkenyl, C₁-C₃ perfluoroalkyl, -O-C₁-C₃-alkyl, -O-C₁-C₃-alkenyl, -CN, C₆-C₁₀ aryl, C₇-C₁₀ arylalkyl, wherein the alkyl groups of said C₁-C₃ alkyl and said O-C₁-C₃-alkyl groups can be substituted by a C₆-C₁₀ heteroaryl group.

4. A compound according to any of the previous claims wherein Ar is a phenyl group.

5. A compound according to any of claims 1 to 3 wherein Ar is selected from the group consisting of a furane, a thiophene and a phenyl substituted by ―F, -Cl, -Br or ―I.

6. A compound according to any of the previous claims wherein n is cero.

7. A compound according to any of the previous claims wherein R₁ is hydrogen.

8. A compound according to any previous claims which is selected from the group consisting of: 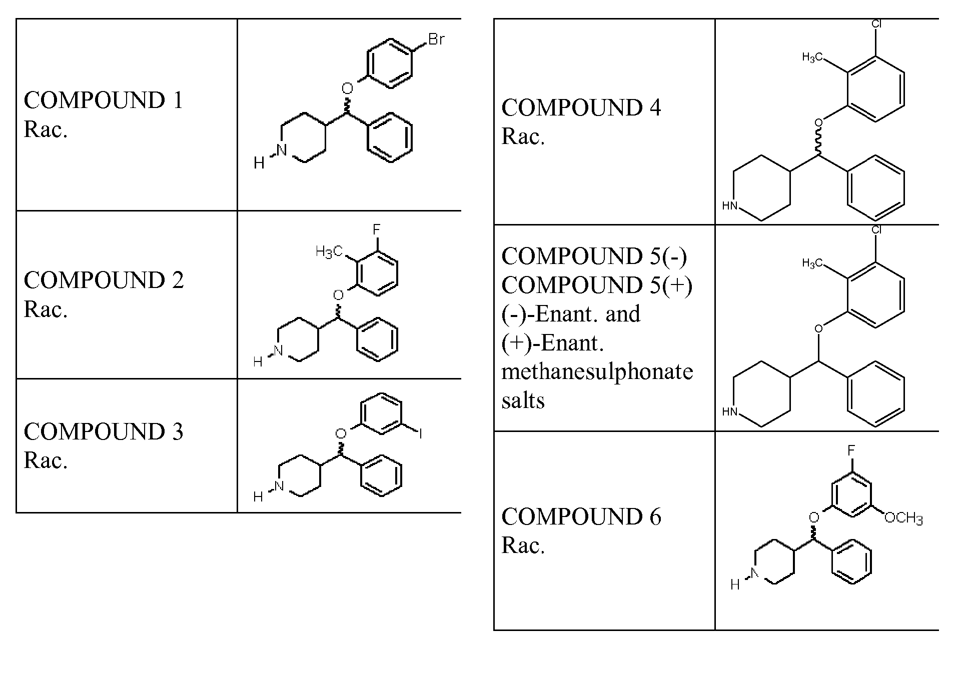 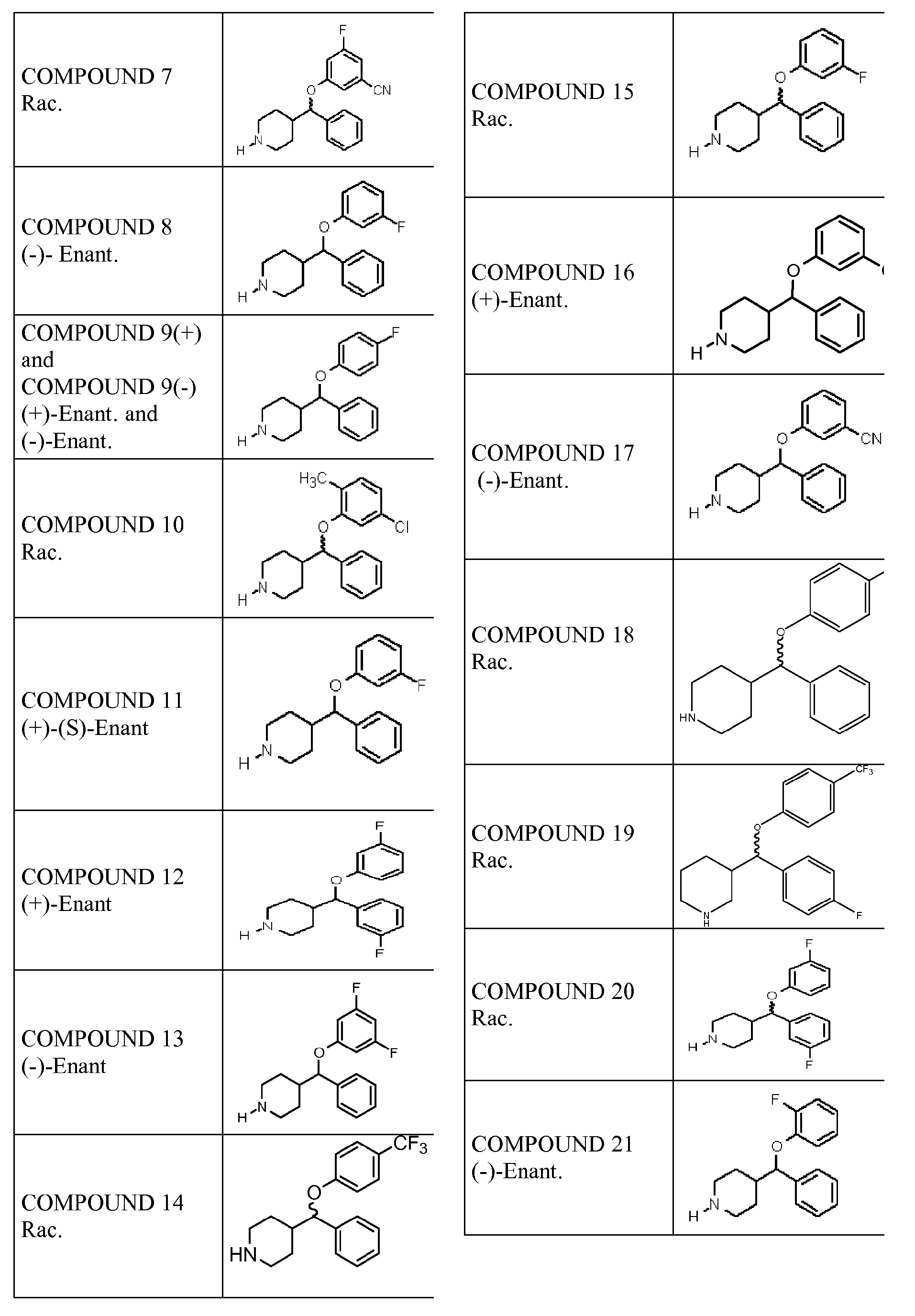 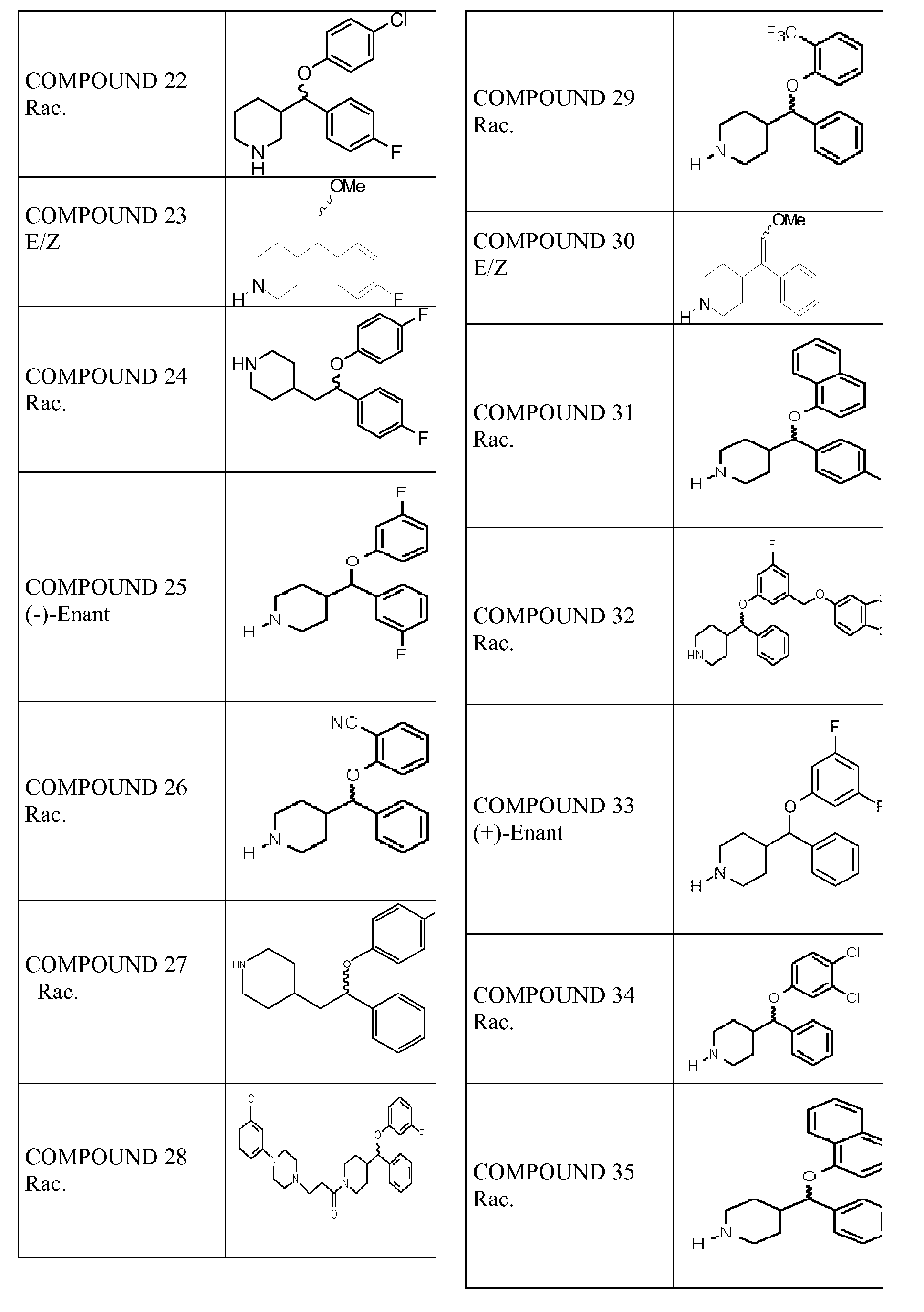 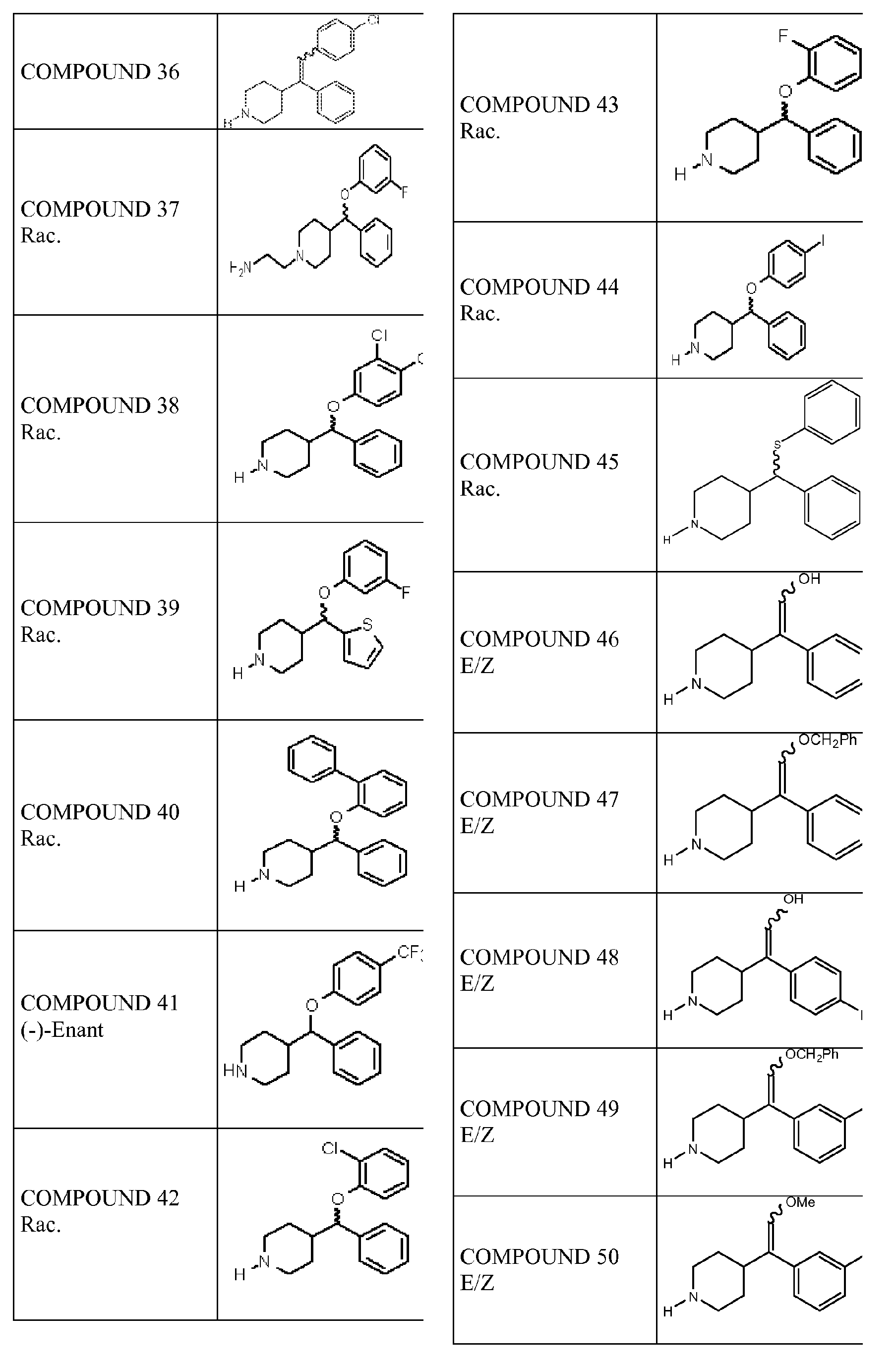 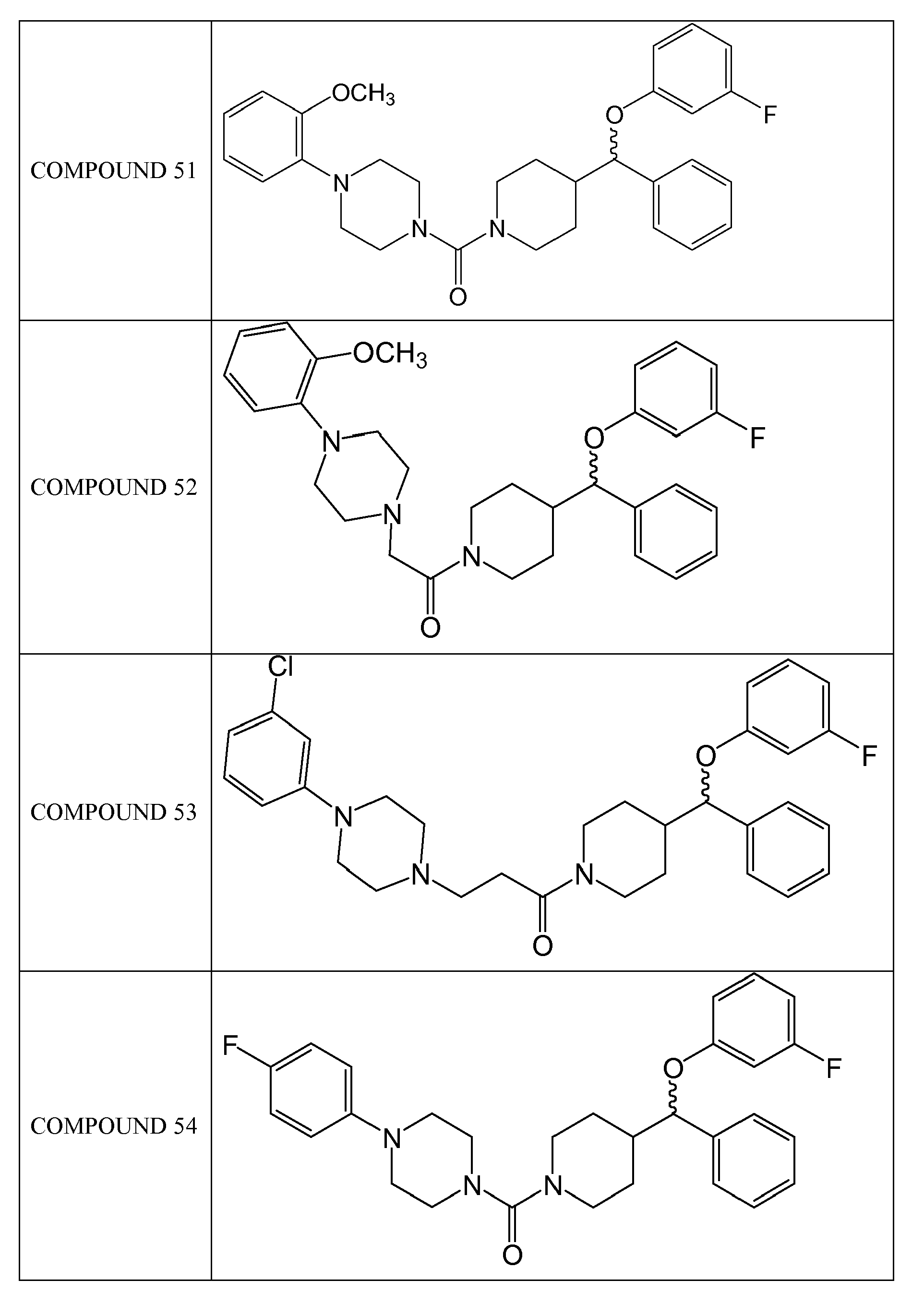 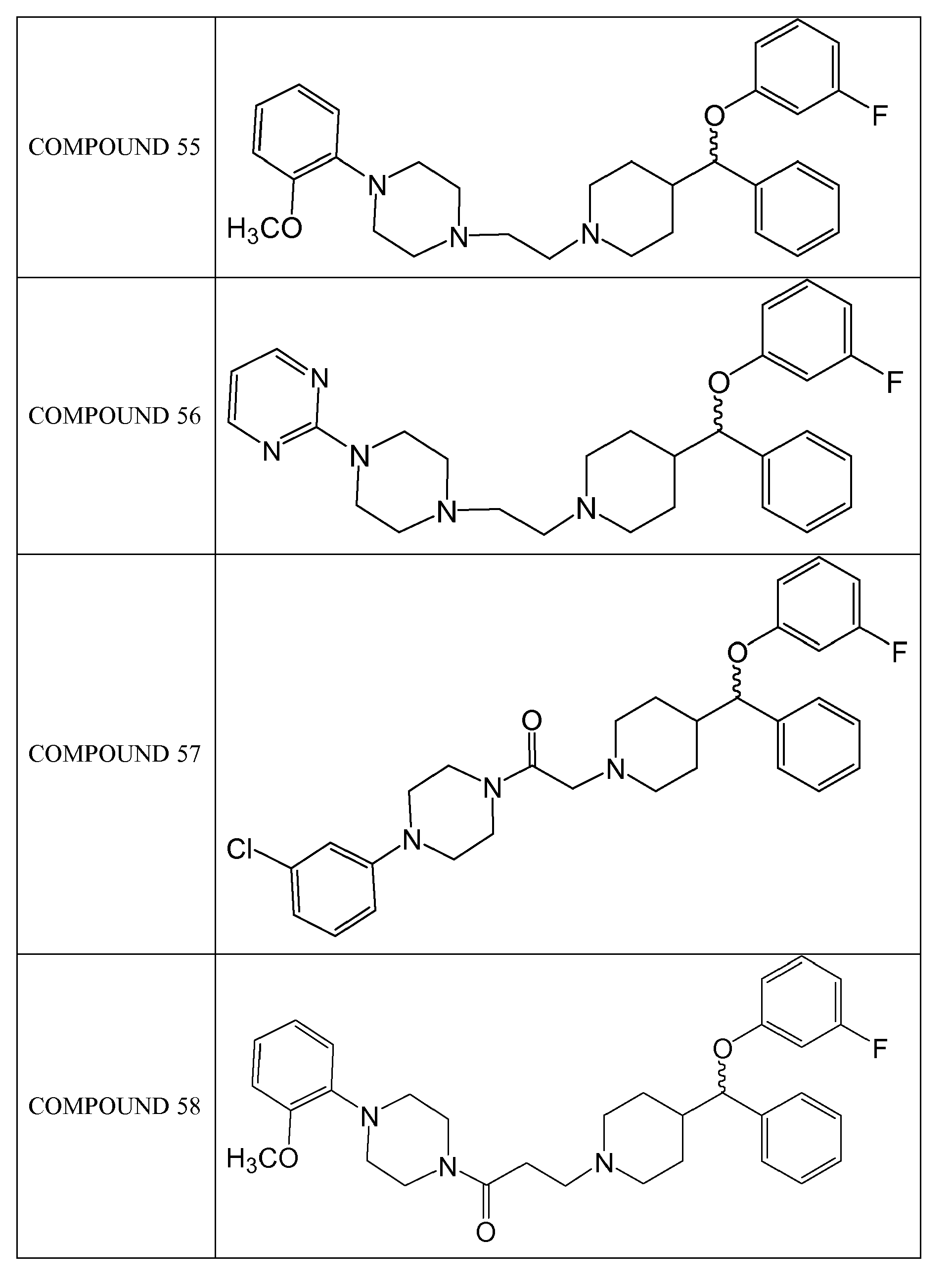 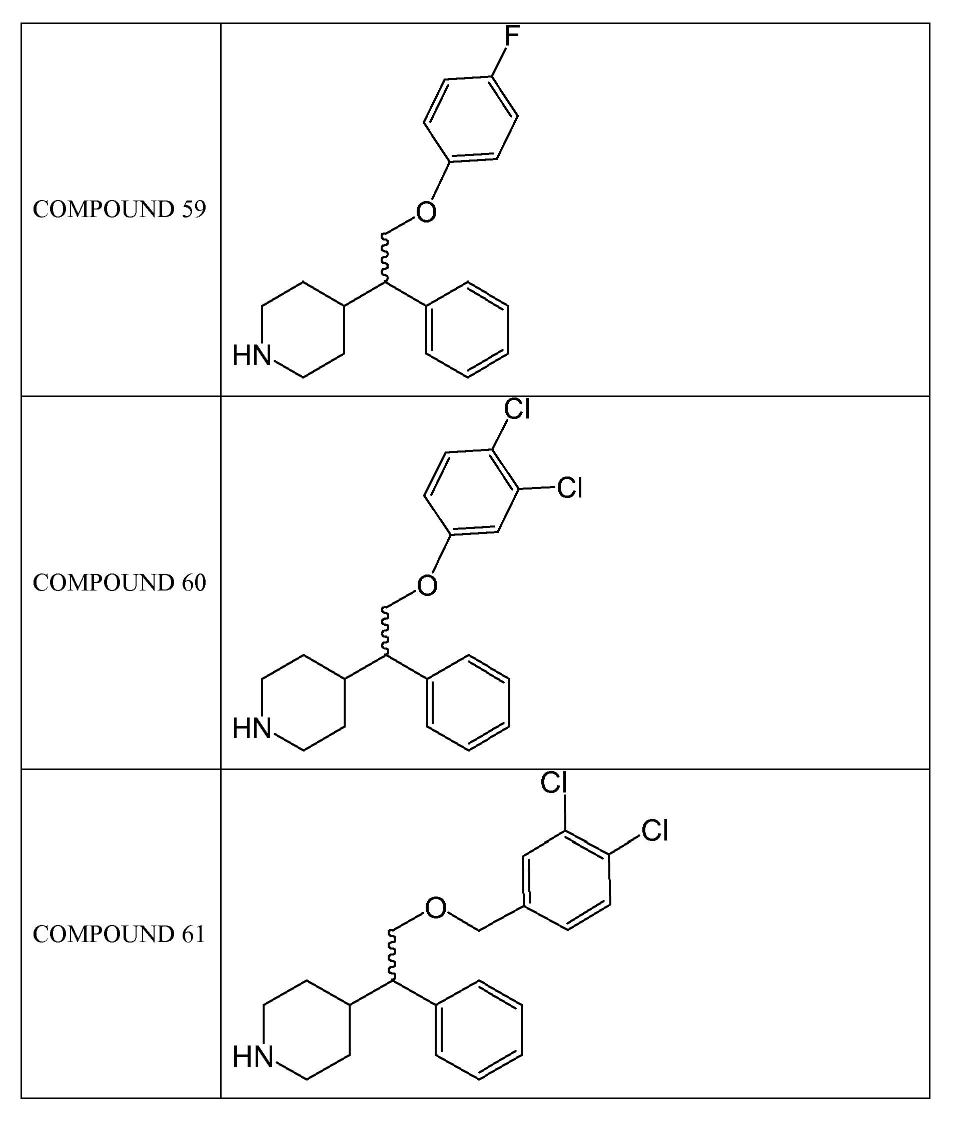 or a pharmaceutically acceptable salt, stereoisomer and/or solvate thereof.

9. A compound of formula (II), of formula (III), of formula (IV), or of formula (V) 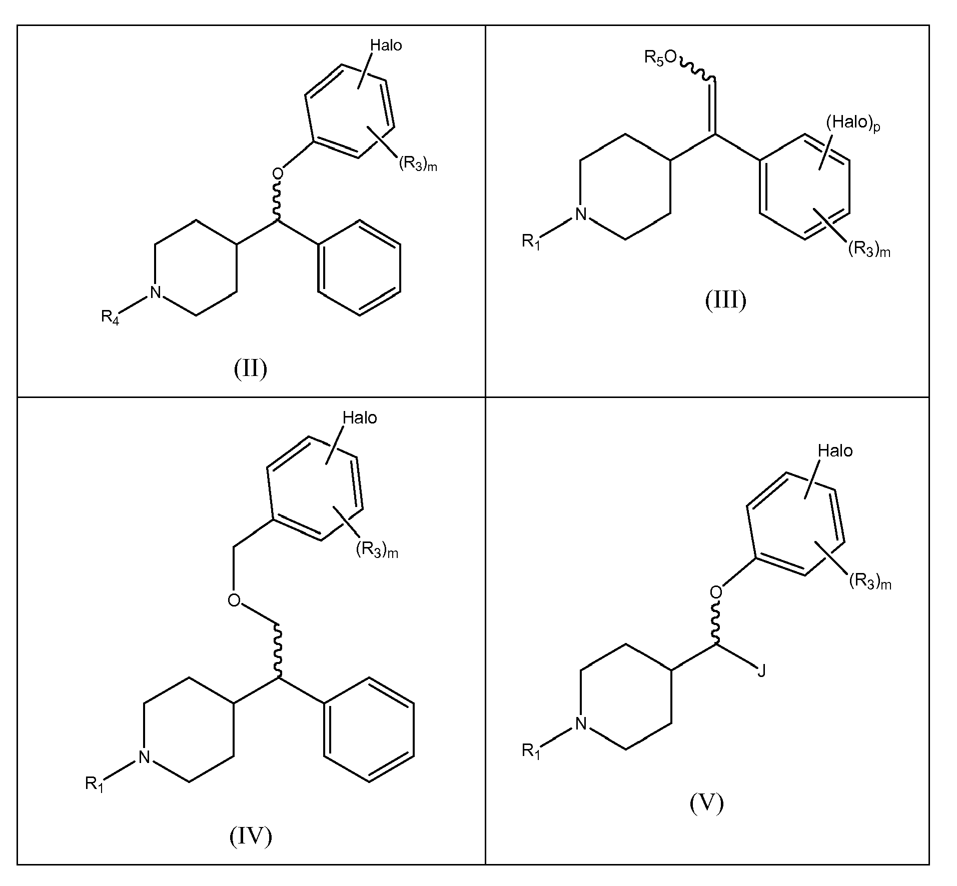 wherein in each case where appropriate
R₁ as defined in any of the previous claims, preferably wherein R₁ is hydrogen or a C₁-C₆ alkyl group optionally substituted by -NH₂, -SH, -OH or C₃-C₈ cycloalkyl;
Ar is an optionally substituted phenyl or an optionally substituted 5- or 6-membered heteroaryl group;
R₄ is -(CH₂)_{w}-(C=O)_{y}―(CH₂)ₓ-R₂, wherein R₂ is a heterocyclyl group optionally substituted by a substituted or unsubstituted phenyl, or by a substituted or
unsubstituted 5- or 6-membered heteroaryl group; w is an integer selected from 0, 1, 2 or 3; x is an integer selected from 0, 1, 2 or 3; and y is 0 or 1;
HeteAryl is an optionally substituted 5- or 6-membered heteroaryl group;
Halo is ―F, -Cl, -Br or ―I;
R₃ is selected from the group consisting of hydrogen, ―F, -Cl, -Br, ―I, C₁-C₃ alkyl,
C₁-C₃ alkenyl, C₁-C₃ perfluoroalkyl, -O-C₁-C₃-alkyl, -O-C₁-C₃-alkenyl, -CN; and
m is 1, 2 or 3; and
p is 0 or 1
or a pharmaceutically acceptable salt, stereoisomer and/or solvate thereof

10. A compound of formula (III), (IV), or (V) according to claim 9 wherein R₃ is hydrogen.

11. A compound of formula (V) according to claim 9 wherein HeteAryl is furan or thiofen.

12. A compound selected from the group consisting of: 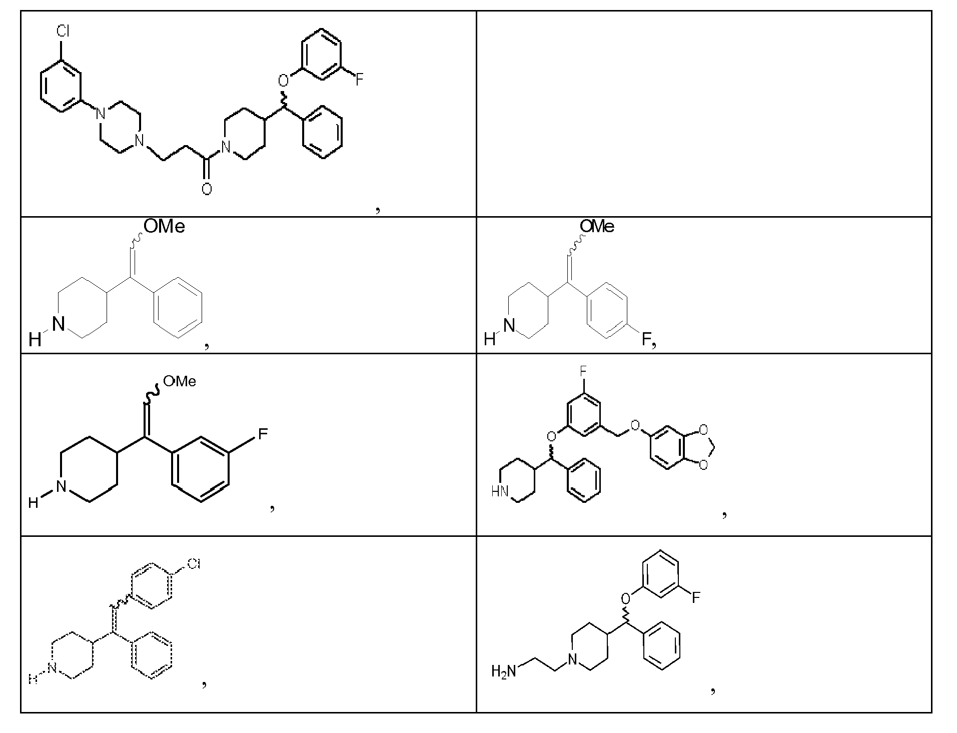 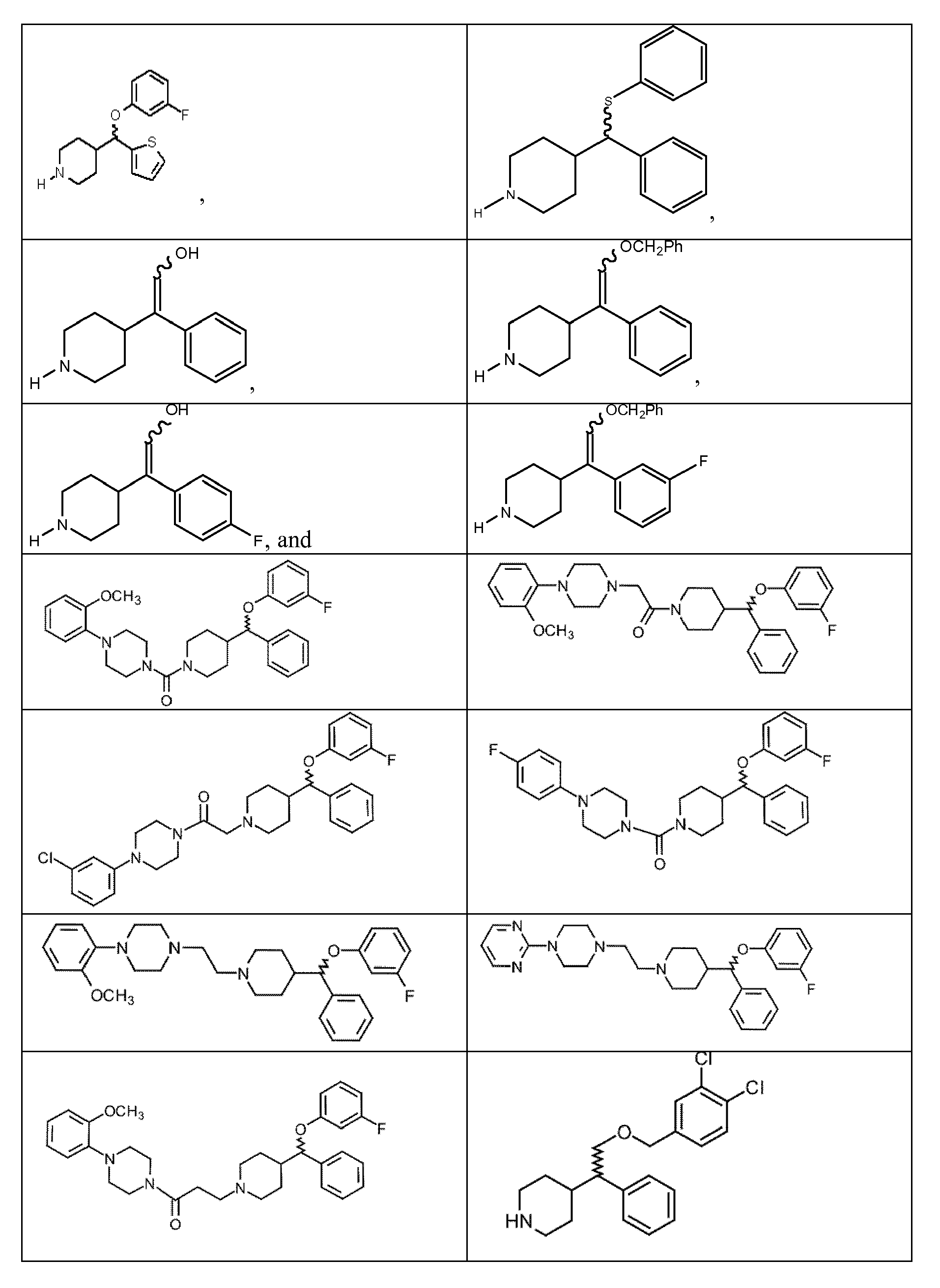 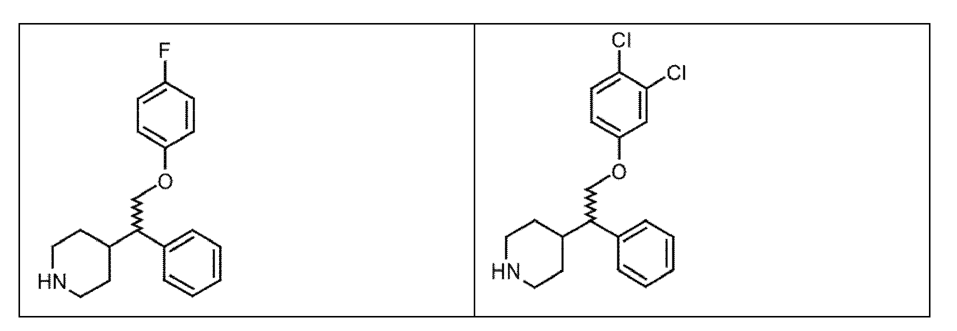 or a pharmaceutically acceptable salt, stereoisomer and/or solvate thereof.

13. A pharmaceutical composition comprising a compound of formula (II), (III), (IV), or (V) as defined in any of claims 9 to 11, or a compound as defined in claim 12, or a pharmaceutically acceptable salt, stereoisomer and/or solvate thereof, and at least one pharmaceutically acceptable carrier.

14. A compound of formula (II), (III), (IV), or (V) as defined in any of claims 9 to 11, or a compound as defined in claim 12, or a pharmaceutically acceptable salt, stereoisomer and/or solvate thereof for use as a medicament.

15. A compound of formula (II), (III), (IV), or (V) as defined in any of claims 9 to 11, or a compound as defined in claim 12, or a pharmaceutically acceptable salt, stereoisomer and/or solvate thereof for use in a method to treat or ameliorate amyloid or tau pathologies or symptoms thereof.
